# EUROPEAN PATENT APPLICATION

(11) **EP 4 403 213 A2**
(43) Date of publication of application: **24.07.2024**
(21) Application number: 24160083.2
(22) Date of filing: 13.03.2015
(51) Int. Cl.: A61P 9/00

(54) **METHODS FOR REDUCING CARDIOVASCULAR RISK**

(30) Priority: 17.03.2014 US 201461954094 P; 16.07.2014 US 201462025400 P; 28.08.2014 US 201462043182 P; 26.02.2015 EP 15305293
(62) Divisional of application: 15712241.7
(71) Applicant: Sanofi Biotechnology, 94250 Gentilly (FR); Regeneron Pharmaceuticals, Inc., Tarrytown, NY 10591 (US)
(72) Inventor: BESSAC, Laurence, 75008 Paris (FR); HANOTIN, Corinne, 75008 Paris (FR); PORDY, Robert, C, Tarrytown, 10591 (US); SASIELA, William, J., Tarrytown, 10591 (US); SCHWARTZ, Gregory, G., Denver, 80220 (US); STEG, Philippe Gabriel, 75877 Paris (FR)
(74) Representative: Zwicker, Jörk

(57) **Abstract**

The present invention provides methods for treating diseases and disorders that are associated with elevated levels of lipids and lipoproteins. The methods of the present invention comprise administering to a high cardiovascular risk patient a pharmaceutical composition comprising a PCSK9 inhibitor. In certain embodiments, the PCSK9 inhibitor is an anti-PCSK9 antibody such as the exemplary antibody referred to herein as mAb316P or alirocumab. The methods of the present invention are useful for treating high cardiovascular risk patients with hypercholesterolemia and elevated levels of other atherogenic lipoproteins that are not adequately controlled by maximum tolerated dose statin therapy. In particular, the methods of the present invention are useful for reducing cardiovascular risk and lowering atherogenic lipoproteins in high cardiovascular risk patients within 12 months following an acute coronary syndrome event despite a maximum tolerated dose statin therapy.

## Description

### FIELD OF THE INVENTION

The present invention relates to the field of therapeutic treatments of diseases and disorders that are associated with elevated levels of lipids and lipoproteins. More specifically, the invention relates to the use of PCSK9 inhibitors for reducing cardiovascular risk and lowering atherogenic lipoproteins in high cardiovascular risk patients following acute coronary syndrome despite a maximum tolerated dose statin therapy.

### BACKGROUND

Despite modern therapy including prompt coronary revascularization, dual antiplatelet therapy, and intensive statin treatment, cardiovascular events occur with high frequency following acute coronary syndrome (ACS). Registry data indicates cardiovascular mortality as high as 13% at 5 years, with an overwhelming majority occurring after initial discharge from the hospital. Patients with recent acute coronary syndrome (ACS) are at very high risk for suffering recurrent coronary events in the near term. In approximately 10% of patients with ACS, cardiovascular death, recurrent myocardial infarction, or stroke occur within 1 year. Based on the results of large clinical trials, early intensive statin therapy has become formally endorsed as a treatment recommendation for patients with ACS. Both epidemiological and pharmacological intervention trials have demonstrated a strong and linear relationship between the levels of low-density lipoprotein cholesterol (LDL-C) and cardiovascular (CV) events. However, many high CV risk patients cannot achieve such levels with currently available lipid lowering drugs. Furthermore, a significant number of high-risk patients even fail to achieve their recommended LDL-C target levels and most CV events are actually not prevented, leaving a substantial "residual risk" for patients. Thus, additional pharmacologic therapies for the prevention of coronary heart disease (CHD) remain essential, particularly for high-risk patients with ACS.

### BRIEF SUMMARY OF THE INVENTION

The present invention provides methods for reducing cardiovascular risk and lowering atherogenic lipoproteins in high cardiovascular risk patients following acute coronary syndrome despite a maximum tolerated dose statin therapy. In particular, the methods of the present invention are useful for reducing cardiovascular risk and/or events.

One embodiment of the present invention provides a method for reducing cardiovascular risk in a high cardiovascular risk patient within 12 months following an acute coronary syndrome (ACS) event comprising administering one or more doses of a proprotein convertase subtilisin/kexin type 9 (PCSK9) inhibitor to the patient, wherein the patient exhibits inadequate control of atherogenic lipoproteins despite steady state treatment with a maximum tolerated dose statin therapy in the absence of the PCSK9 inhibitor.

One embodiment of the present invention provides a method for reducing cardiovascular events in a high cardiovascular risk patient within 12 months following an acute coronary syndrome (ACS) event comprising administering one or more doses of a proprotein convertase subtilisin/kexin type 9 (PCSK9) inhibitor to the patient, wherein the patient exhibits inadequate control of atherogenic lipoproteins despite steady state treatment with a maximum tolerated dose statin therapy in the absence of the PCSK9 inhibitor.

One embodiment of the present invention provides a method for reducing cardiovascular events in a high cardiovascular risk patient following an acute coronary syndrome (ACS) event comprising administering one or more doses of a proprotein convertase subtilisin/kexin type 9 (PCSK9) inhibitor to the patient.

One embodiment of the present invention provides a method for reducing cardiovascular risk in a high cardiovascular risk patient following an acute coronary syndrome (ACS) event comprising administering one or more doses of a proprotein convertase subtilisin/kexin type 9 (PCSK9) inhibitor to the patient.

According to one aspect, the methods of the present invention comprise administering one or more doses of a PCSK9 inhibitor to a high cardiovascular risk patient for reducing cardiovascular risk and lowering atherogenic lipoproteins in the patient within 12 months following an acute coronary syndrome event despite a maximum tolerated dose statin therapy (i.e., elevated lipids and lipoproteins that are not adequately controlled by maximum tolerated dose statin therapy in the absence of a PCSK9 inhibitor). According to certain embodiments of the present invention, the PCSK9 inhibitor is administered to the high cardiovascular risk patient as an add-on therapy to the patient's existing statin therapy.

According to another aspect, the methods of the present invention comprise selecting a high cardiovascular risk patient who is on a therapeutic regimen comprising a daily dose of a statin (e.g., a maximum tolerated dose statin therapy), and administering to the patient one or more doses of a PCSK9 inhibitor in combination with (i.e., "on top of") the statin therapy.

According to one aspect, the invention includes a method for reducing cardiovascular risk in a high cardiovascular risk patient within 12 months following an acute coronary syndrome (ACS) event comprising administering one or more doses of a proprotein convertase subtilisin/kexin type 9 (PCSK9) inhibitor to the patient, wherein the patient exhibits inadequate control of atherogenic lipoproteins despite steady state treatment with a maximum tolerated dose statin therapy in the absence of the PCSK9 inhibitor.

In some aspects, the term reducing cardiovascular risk means reducing the time to first occurrence of coronary heart disease death, acute myocardial infarction, hospitalization for unstable angina, or ischemic stroke.

In some aspects, the term ACS event is defined by: 1) unstable symptoms of myocardial ischemia occurring at rest or minimal exertion within 72 hours of an unscheduled hospital admission, due to presumed or proven obstructive coronary disease; and 2) at least one of the following: a) elevated cardiac biomarkers consistent with acute myocardial infarction, or b) resting ECG changes consistent with ischemia or infarction along with additional evidence of obstructive coronary disease from regional perfusion imaging or wall motion abnormalities, epicardial coronary stenosis ≥70% by angiography, or need for coronary revascularization related to the event.

In some aspects, the PCSK9 inhibitor is an antibody or an antigen-binding fragment thereof that specifically binds PCSK9. In some aspects, the antibody or antigen binding fragment thereof comprises the heavy and light chain complementarity determining regions (CDRs) of a heavy chain variable region/light chain variable region (HCVR/LCVR) amino acid sequence pair selected from the group consisting of SEQ ID NOs: 1/6 and 11/15. In some aspects, the antibody or antigen-binding fragment thereof comprises heavy and light chain CDR amino acid sequences having SEQ ID NOs: 12, 13, 14, 16, 17, and 18. In alternative aspects, the antibody or antigen-binding fragment thereof comprises an HCVR having the amino acid sequence of SEQ ID NO: 11 and an LCVR having the amino acid sequence of SEQ ID NO:15. In some aspects, the antibody or antigen-binding fragment thereof comprises heavy and light chain CDR amino acid sequences having SEQ ID NOs: 2, 3, 4, 7, 8, and 10. In alternative aspects, the antibody or antigen-binding fragment thereof comprises an HCVR having the amino acid sequence of SEQ ID NO:1 and an LCVR having the amino acid sequence of SEQ ID NO:6. In other aspects, the antibody or antigen-binding fragment thereof binds to the same epitope on PCSK9 as an antibody comprising heavy and light chain CDR amino acid sequences having SEQ ID NOs: 12, 13, 14, 16, 17, and 18; or SEQ ID NOs: 2, 3, 4, 7, 8, and 10. In yet other aspects, the antibody or antigen-binding fragment thereof competes for binding to PCSK9 with an antibody comprising heavy and light chain CDR amino acid sequences having SEQ ID NOs: 12, 13, 14, 16, 17, and 18; or SEQ ID NOs: 2, 3, 4, 7, 8, and 10.

In some aspects, the antibody or antigen-binding fragment thereof that specifically binds PCSK9 is administered to the patient at a dose of about 75 mg at a frequency of once every two weeks. In other aspects, the about 75 mg dose is maintained if the patient's LDL-C measured after two doses is <50 mg/dL. In yet other aspects, the about 75 mg dose is discontinued if the patient's LDL-C measured after two doses remains ≥50 mg/dL, and the antibody or antigen-binding fragment thereof that specifically binds PCSK9 is subsequently administered to the patient at a dose of about 150 mg at a frequency of once every two weeks. In further aspects, the about 150 mg dose is discontinued if the patient's LDL-C for any two consecutive measurements is <25 mg/dL, and the antibody or antigen-binding fragment thereof that specifically binds PCSK9 is subsequently administered to the patient at a dose of about 75 mg at a frequency of once every two weeks.

In some aspects, the PCSK9 inhibitor is administered to the patient in combination with the maximum tolerated dose statin therapy. In other aspects, the maximum tolerated dose statin therapy comprises a daily dose of about 40 mg to about 80 mg of atorvastatin. In yet other aspects, the maximum tolerated dose statin therapy comprises a daily dose of about 20 mg to about 40 mg of rosuvastatin.

In some aspects, the patient, prior to or at the time of administration of the PCSK9 inhibitor, exhibits inadequate control of atherogenic lipoproteins defined as: 1) a serum low-density lipoprotein cholesterol (LDL-C) level of ≥70 mg/dL; 2) non-high-density lipoprotein cholesterol ≥100 mg/dL; or 3) apolipoprotein B ≥80 mg/dL.

In some aspects, the steady state treatment is treatment for at least two weeks.

Other embodiments of the present invention will become apparent from a review of the ensuing detailed description.

### BRIEF DESCRIPTION OF THE FIGURES

Figure 1 is a graphic representation of the design of the ODYSSEY LONG TERM study.
Figure 2 is a graphic representation of the study design for ODYSSEY LONG TERM. Phone call visits are indicated in italics, and continue every 4 weeks between on-site visits until the end of the double-blind treatment period visit.
Figure 3 is a graph showing LS mean (SE) calculated LDL-C for placebo and alirocumab at each time point in the ODYSSEY LONG TERM study up to Week 52. The values indicted on the graph are the LS mean % change from baseline to week 24 and week 52.

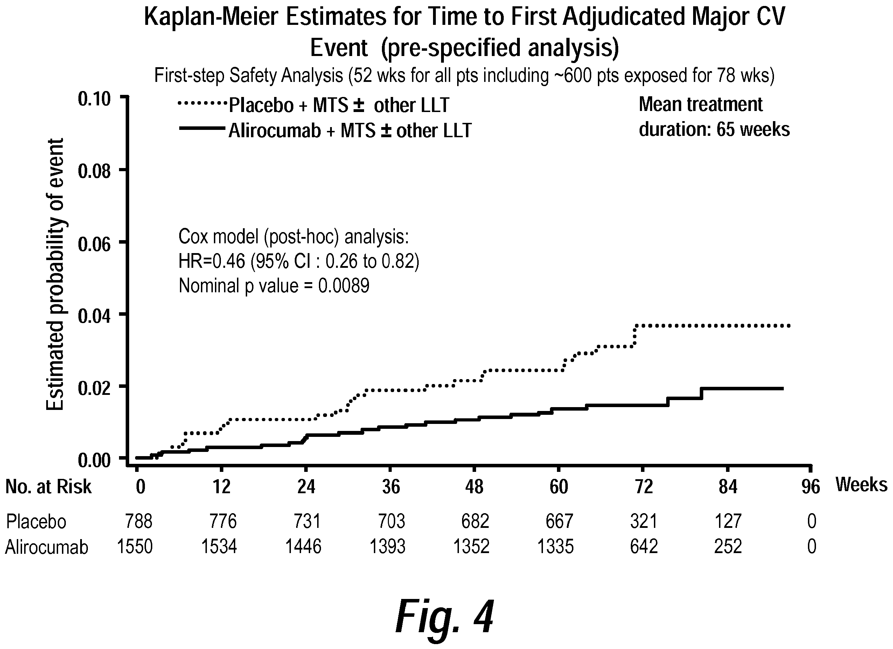

Figure 4 is graph of Kaplan-Meier estimates for time to first adjudicated major CV event for the ODYSSEY LONG TERM study at the time of the pre-specified analysis.
Figure 5 is graph showing LS mean (SE) calculated non-HDL-C, ApoB and Lp(a) levels for the placebo and alirocumab groups at 24 weeks in the ODYSSEY LONG TERM study.
Figure 6 is a graph of a *post hoc* analysis of a subgroup of adjudicated MACE (ODYSSEY OUTCOMES endpoint), showing the Kaplan-Meier estimates for time to first positively adjudicated CV event during the TEAE period (at study completion)
Figure 7 is a graph of a *post hoc* analysis of a subgroup of adjudicated MACE (ODYSSEY OUTCOMES endpoint), showing the Kaplan-Meier estimates for time to first positively adjudicated CV event during the TEAE period in a pool of Phase 3 placebo-controlled studies.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 1%. For example, as used herein, the expression "about 100" includes 99 and 101 and all values in between (e.g., 99.1, 99.2, 99.3, 99.4, etc.).

Although any methods and materials similar or equivalent to those described herein can be used in the practice of the present invention, the preferred methods and materials are now described. All publications mentioned herein are incorporated herein by reference in their entirety.

### Hypercholesterolemia And Other Atherogenic Lipoproteins Not Adequately Controlled by Maximum Tolerated Dose Statin Therapy

The present invention relates generally to methods and compositions for treating high cardiovascular risk patients who have hypercholesterolemia that is not adequately controlled by statins, *i.e*., hypercholesterolemia not adequately controlled by a therapeutic regimen comprising a daily maximum tolerated dose of a statin. As used herein, the expression "not adequately controlled", in reference to hypercholesterolemia, means that the patient's serum low-density lipoprotein cholesterol (LDL-C) concentration, total cholesterol concentration, and/or triglyceride concentration is not reduced to a recognized, medically-acceptable level (taking into account the patient's relative risk of coronary heart disease) after at least 4 weeks on a therapeutic regimen comprising a stable daily dose of a statin. For example, "a patient with hypercholesterolemia that is not adequately controlled by a statin" includes a patient or patients with a serum LDL-C concentration of greater than or equal to about 70 mg/dL, 100 mg/dL, 130 mg/dL, 140 mg/dL, or more (depending on the patient's underlying risk of heart disease) after the patient has been on a stable daily statin regimen for at least 4 weeks.

According to certain embodiments, the high cardiovascular risk patient who is treatable by the methods of the present invention has hypercholesterolemia (e.g., a serum LDL-C concentration of greater than or equal to 70 mg/dL) despite taking a stable daily dose of a statin (with or without other lipid modifying therapy) for at least 4 weeks, 5 weeks, 6 weeks, or more. In certain embodiments, the high cardiovascular risk patient's hypercholesterolemia is inadequately controlled by a maximum tolerated dose statin therapy.

The present invention also relates generally to methods and compositions for treating high cardiovascular risk patients who have elevated levels of atherogenic lipoproteins that are not adequately controlled by statins, i.e., elevated levels of atherogenic lipoproteins not adequately controlled by a therapeutic regimen comprising a daily maximum tolerated dose of a statin. As used herein, the expression "not adequately controlled", in reference to atherogenic lipoproteins, means that the patient's serum low-density lipoprotein cholesterol (LDL-C) concentration, non-high-density lipoprotein cholesterol, and/or apolipoprotein B concentration are not reduced to a recognized, medically-acceptable level (taking into account the patient's relative risk of coronary heart disease) after at least 4 weeks on a therapeutic regimen comprising a stable daily dose of a statin. For example, "a patient with elevated levels of atherogenic lipoproteins that are not adequately controlled by a statin" includes a patient or patients with a serum LDL-C concentration of greater than or equal to about 70 mg/dL, 100 mg/dL, 130 mg/dL, 140 mg/dL, or more (depending on the patient's underlying risk of heart disease); a non-high-density lipoprotein cholesterol concentration of greater than or equal to about 100 mg/dL; or an apolipoprotein B concentration of greater than or equal to about 80 mg/dL after the patient has been on a stable daily statin regimen for at least 4 weeks.

According to certain embodiments, the high cardiovascular risk patient who is treatable by the methods of the present invention has elevated levels of atherogenic lipoproteins (e.g., a serum LDL-C concentration of greater than or equal to 70 mg/dL) despite taking a stable daily dose of a statin (with or without other lipid modifying therapy) for at least 4 weeks, 5 weeks, 6 weeks, or more. In certain embodiments, the high cardiovascular risk patient's elevated levels of atherogenic lipoproteins are inadequately controlled by a maximum tolerated dose statin therapy.

As used herein, "maximum tolerated dose statin therapy" means a therapeutic regimen comprising the administration of daily dose of a statin that is the maximally tolerated dose for a particular patient. Maximally tolerated dose means the highest dose of statin that can be administered to a patient without causing unacceptable adverse side effects in the patient. Maximum tolerated dose statin therapy includes but is not limited to, e.g., 40-80 mg of atorvastatin daily, or 20-40 mg of rosuvastatin daily.

The present invention also includes methods for treating high cardiovascular risk patients with hypercholesterolemia and elevated levels of other atherogenic lipoproteins that are not adequately controlled by maximum tolerated dose statin therapy comprising daily administration of other statins such as cerivastatin, pitavastatin, fluvastatin, lovastatin, and pravastatin.

### Patient Selection

The present invention includes methods and compositions useful for treating high cardiovascular risk patients who have hypercholesterolemia and elevated levels of other atherogenic lipoproteins that are not adequately controlled by a daily maximum tolerated dose therapeutic statin regimen.

In certain embodiments of the present invention, the antibody or antigen-binding fragment thereof is administered as an adjunct to diet.

In some embodiments, the antibody or antigen-binding fragment thereof is administered for the reduction of cardiovascular events in patients with recent acute coronary syndrome.

In some embodiments, the antibody or antigen-binding fragment thereof is administered for the reduction of cardiovascular risk in patients with recent acute coronary syndrome.

In some embodiments, the antibody or antigen-binding fragment thereof is either in combination with a statin or as monotherapy including in patients who cannot tolerate statins.

In some embodiments, the antibody or antigen-binding fragment thereof is administered as a subcutaneous injection into the thigh, abdomen, or upper arm using a single-use pre-filled pen or single-use pre-filled syringe. The injection site can be rotated with each injection. The antibody of antigen-binding fragment thereof should not be injected into areas of active skin disease or injury such as sunburns, skin rashes, inflammation, or skin infections.

The high cardiovascular risk patients who are treatable by the methods of the present invention were hospitalized for ACS defined by unstable symptoms of myocardial ischemia occurring at rest or minimal exertion within 72 hours of an unscheduled hospital admission, due to presumed or proven obstructive coronary disease. In addition, a qualifying ACS event required at least one of the following criteria to be fulfilled: elevated cardiac biomarkers consistent with acute myocardial infarction, or resting ECG changes consistent with ischemia or infarction along with additional evidence of obstructive coronary disease from regional perfusion imaging or wall motion abnormalities, epicardial coronary stenosis ≥70% by angiography, or need for coronary revascularization related to the event.

Qualifying patients must have demonstrated inadequate control of atherogenic lipoproteins despite steady state (at least 2 weeks) treatment with atorvastatin 40-80 mg daily, rosuvastatin 20-40 mg daily, or the maximum tolerated dose of one of these agents. Inadequate control of atherogenic lipoproteins was defined by at least one of the following: LDL-C ≥70 mg/dL (1.81 mmol/L), non-high density lipoprotein cholesterol (non-HDL-C) ≥100 mg/dL (2.59 mmol/L), or apolipoprotein B ≥80 mg/dL (0.8 mmol/L).

According to certain embodiments, the high cardiovascular risk patient may be selected on the basis of having one or more additional risk factors selected from the group consisting of age (e.g., older than 40, 45, 50, 55, 60, 65, 70, 75, or 80 years), race, national origin, gender (male or female), exercise habits (e.g., regular exerciser, non-exerciser), other preexisting medical conditions (e.g., type-II diabetes, high blood pressure, etc.), and current medication status (e.g., currently taking beta blockers, niacin, ezetimibe, fibrates, omega-3 fatty acids, bile acid resins, etc.).

According to the present invention, high cardiovascular risk patients may be selected on the basis of a combination of one or more of the foregoing selection criteria or therapeutic characteristics.

### Administration of a PCSK9 Inhibitor as Add-On Therapy to Maximum Tolerated Dose Statin Therapy

The present invention includes methods wherein a high cardiovascular risk patient with hypercholesterolemia and elevated levels of other atherogenic lipoproteins that are not adequately controlled by a stable daily maximum tolerated dose therapeutic statin regimen in the absence of a PCSK9 inhibitor is administered a PCSK9 inhibitor according to a particular dosing amount and frequency, and wherein the PCSK9 inhibitor is administered as an add-on to the patient's therapeutic statin regimen. For example, according to certain embodiments, if a high cardiovascular risk patient has hypercholesterolemia and elevated levels of other atherogenic lipoproteins that are not adequately controlled despite being on a stable daily maximum tolerated dose therapeutic statin regimen comprising, e.g., 40-80 mg of atorvastatin, the high cardiovascular risk patient may be administered a PCSK9 inhibitor at a particular amount and dosing interval while the patient continues his or her stable daily therapeutic statin regimen.

The methods of the present invention include add-on therapeutic regimens wherein the PCSK9 inhibitor is administered as add-on therapy to the same stable daily maximum tolerated dose therapeutic statin regimen (i.e., same dosing amount of statin) that the high cardiovascular risk patient was on prior to receiving the PCSK9 inhibitor. In other embodiments, the PCSK9 inhibitor is administered as add-on therapy to a daily maximum tolerated dose therapeutic statin regimen comprising a statin in an amount that is more than or less than the dose of statin the patient was on prior to receiving the PCSK9 inhibitor. For example, after starting a therapeutic regimen comprising a PCSK9 inhibitor administered at a particular dosing frequency and amount, the daily dose of statin administered or prescribed to the patient may (a) stay the same, (b) increase, or (c) decrease (e.g., up-titrate or down-titrate) in comparison to the daily statin dose the high cardiovascular risk patient was taking before starting the PCSK9 inhibitor therapeutic regimen, depending on the therapeutic needs of the patient.

### Therapeutic Efficacy

The methods of the present invention will result in the reduction in serum levels of one or more lipid components selected from the group consisting of LDL-C, ApoB100, non-HDL-C, total cholesterol, VLDL-C, triglycerides, Lp(a) and remnant cholesterol. For example, according to certain embodiments of the present invention, administration of a pharmaceutical composition comprising a PCSK9 inhibitor to a high cardiovascular risk patient with hypercholesterolemia or elevated levels of other atherogenic lipoproteins that are not adequately controlled by a stable daily maximum tolerated dose therapeutic statin regimen, (e.g., administration of the PCSK9 inhibitor on top of the high cardiovascular risk patient's maximum tolerated dose statin therapy) will result in a mean percent reduction from baseline in serum low density lipoprotein cholesterol (LDL-C) of at least about 25%, 30%, 40%, 50%, 60%, or greater; a mean percent reduction from baseline in ApoB100 of at least about 25%, 30%, 40%, 50%, 60%, or greater; a mean percent reduction from baseline in non-HDL-C of at least about 25%, 30%, 40%, 50%, 60%, or greater; a mean percent reduction from baseline in total cholesterol of at least about 10%, 15%, 20%, 25%, 30%, 35%, or greater; a mean percent reduction from baseline in VLDL-C of at least about 5%, 10%, 15%, 20%, 25%, 30%, or greater; a mean percent reduction from baseline in triglycerides of at least about 5%, 10%, 15%, 20%, 25%, 30%, 35% or greater; and/or a mean percent reduction from baseline in Lp(a) of at least about 5%, 10%, 15%, 20%, 25%, or greater.

### PCSK9 Inhibitors

The methods of the present invention comprise administering to a high cardiovascular risk patient a therapeutic composition comprising a PCSK9 inhibitor. As used herein, a "PCSK9 inhibitor" is any agent which binds to or interacts with human PCSK9 and inhibits the normal biological function of PCSK9 *in vitro* or *in vivo.* Non-limiting examples of categories of PCSK9 inhibitors include small molecule PCSK9 antagonists, peptide-based PCSK9 antagonists (e.g., "peptibody" molecules), and antibodies or antigen-binding fragments of antibodies that specifically bind human PCSK9.

The term "human proprotein convertase subtilisin/kexin type 9" or "human PCSK9" or "hPCSK9", as used herein, refers to PCSK9 having the nucleic acid sequence shown in SEQ ID NO:197 and the amino acid sequence of SEQ ID NO:198, or a biologically active fragment thereof.

The term "antibody", as used herein, is intended to refer to immunoglobulin molecules comprising four polypeptide chains, two heavy (H) chains and two light (L) chains inter-connected by disulfide bonds, as well as multimers thereof (e.g., IgM). Each heavy chain comprises a heavy chain variable region (abbreviated herein as HCVR or VH) and a heavy chain constant region. The heavy chain constant region comprises three domains, CH1, CH2 and CH3. Each light chain comprises a light chain variable region (abbreviated herein as LCVR or VL) and a light chain constant region. The light chain constant region comprises one domain (CL1). The VH and VL regions can be further subdivided into regions of hypervariability, termed complementarity determining regions (CDRs), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. In different embodiments of the invention, the FRs of the anti-PCSK9 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (e.g. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a VH domain associated with a VL domain, the VH and VL domains may be situated relative to one another in any suitable arrangement. For example, the variable region may be dimeric and contain VH-VH, VH-VL or VL-VL dimers. Alternatively, the antigen-binding fragment of an antibody may contain a monomeric VH or VL domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present invention include: (i) VH-CH1; (ii) VH-CH2; (iii) VH-CH3; (iv) VH-CH1-CH2; (v) VH-CH1-CH2-CH3; (vi) VH-CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any configuration of variable and constant domains, including any of the exemplary configurations listed above, the variable and constant domains may be either directly linked to one another or may be linked by a full or partial hinge or linker region. A hinge region may consist of at least 2 (e.g., 5, 10, 15, 20, 40, 60 or more) amino acids which result in a flexible or semi-flexible linkage between adjacent variable and/or constant domains in a single polypeptide molecule. Moreover, an antigen-binding fragment of an antibody of the present invention may comprise a homo-dimer or hetero-dimer (or other multimer) of any of the variable and constant domain configurations listed above in noncovalent association with one another and/or with one or more monomeric VH or VL domain (e.g., by disulfide bond(s)).

As with full antibody molecules, antigen-binding fragments may be monospecific or multispecific (e.g., bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody of the present invention using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies of the invention may nonetheless include amino acid residues not encoded by human germline immunoglobulin sequences (e.g., mutations introduced by random or site-specific mutagenesis in vitro or by somatic mutation in vivo), for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo.

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via interchain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant invention encompasses antibodies having one or more mutations in the hinge, CH2 or CH3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody in situ within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" PCSK9, as used in the context of the present invention, includes antibodies that bind PCSK9 or portion thereof with a KD of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human PCSK9 however may in certain embodiments have cross-reactivity to other antigens, such as PCSK9 molecules from other (non-human) species.

The anti-PCSK9 antibodies useful for the methods of the present invention may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present invention includes methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain embodiments, all of the framework and/or CDR residues within the VH and/or VL domains are mutated back to the residues found in the original germline sequence from which the antibody was derived. In other embodiments, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (i.e., a germline sequence that is different from the germline sequence from which the antibody was originally derived). Furthermore, the antibodies of the present invention may contain any combination of two or more germline mutations within the framework and/or CDR regions, e.g., wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present invention.

The present invention also includes methods involving the use of anti-PCSK9 antibodies comprising variants of any of the HCVR, LCVR, and/or CDR amino acid sequences disclosed herein having one or more conservative substitutions. For example, the present invention includes the use of anti-PCSK9 antibodies having HCVR, LCVR, and/or CDR amino acid sequences with, e.g., 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance", as used herein, refers to an optical phenomenon that allows for the analysis of real-time interactions by detection of alterations in protein concentrations within a biosensor matrix, for example using the BIAcore^{™} system (Biacore Life Sciences division of GE Healthcare, Piscataway, NJ).

The term "KD ", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

According to certain embodiments, the anti-PCSK9 antibody used in the methods of the present invention is an antibody with pH-dependent binding characteristics. As used herein, the expression "pH-dependent binding" means that the antibody or antigen-binding fragment thereof exhibits "reduced binding to PCSK9 at acidic pH as compared to neutral pH" (for purposes of the present disclosure, both expressions may be used interchangeably). For the example, antibodies "with pH-dependent binding characteristics" include antibodies and antigen-binding fragments thereof that bind PCSK9 with higher affinity at neutral pH than at acidic pH. In certain embodiments, the antibodies and antigen-binding fragments of the present invention bind PCSK9 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

According to this aspect of the invention, the anti-PCSK9 antibodies with pH-dependent binding characteristics may possess one or more amino acid variations relative to the parental anti-PCSK9 antibody. For example, an anti-PCSK9 antibody with pH-dependent binding characteristics may contain one or more histidine substitutions or insertions, e.g., in one or more CDRs of a parental anti-PCSK9 antibody. Thus, according to certain embodiments of the present invention, methods are provided comprising administering an anti-PCSK9 antibody which comprises CDR amino acid sequences (e.g., heavy and light chain CDRs) which are identical to the CDR amino acid sequences of a parental anti-PCSK9 antibody, except for the substitution of one or more amino acids of one or more CDRs of the parental antibody with a histidine residue. The anti-PCSK9 antibodies with pH-dependent binding may possess, e.g., 1, 2, 3, 4, 5, 6, 7, 8, 9, or more histidine substitutions, either within a single CDR of a parental antibody or distributed throughout multiple (e.g., 2, 3, 4, 5, or 6) CDRs of a parental anti-PCSK9 antibody. For example, the present invention includes the use of anti-PCSK9 antibodies with pH-dependent binding comprising one or more histidine substitutions in HCDR1, one or more histidine substitutions in HCDR2, one or more histidine substitutions in HCDR3, one or more histidine substitutions in LCDR1, one or more histidine substitutions in LCDR2, and/or one or more histidine substitutions in LCDR3, of a parental anti-PCSK9 antibody.

As used herein, the expression "acidic pH" means a pH of 6.0 or less (e.g., less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

### Preparation of Human Antibodies

Methods for generating human antibodies in transgenic mice are known in the art. Any such known methods can be used in the context of the present invention to make human antibodies that specifically bind to human PCSK9.

Using VELOCIMMUNE^{™} technology (see, for example, US 6,596,541, Regeneron Pharmaceuticals) or any other known method for generating monoclonal antibodies, high affinity chimeric antibodies to PCSK9 are initially isolated having a human variable region and a mouse constant region. The VELOCIMMUNE^{®} technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE^{®} mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc, using standard procedures known to those skilled in the art. The mouse constant regions are replaced with a desired human constant region to generate the fully human antibody of the invention, for example wild-type or modified IgG1 or IgG4. While the constant region selected may vary according to specific use, high affinity antigen-binding and target specificity characteristics reside in the variable region.

In general, the antibodies that can be used in the methods of the present invention possess high affinities, as described above, when measured by binding to antigen either immobilized on solid phase or in solution phase. The mouse constant regions are replaced with desired human constant regions to generate the fully human antibodies of the invention. While the constant region selected may vary according to specific use, high affinity antigen-binding and target specificity characteristics reside in the variable region.

Specific examples of human antibodies or antigen-binding fragments of antibodies that specifically bind PCSK9 which can be used in the context of the methods of the present invention include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs:1 and 11, or a substantially similar sequence thereof having at least 90%, at least 95%, at least 98% or at least 99% sequence identity. Alternatively, specific examples of human antibodies or antigen-binding fragments of antibodies that specifically bind PCSK9 which can be used in the context of the methods of the present invention include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs 37, 45, 53, 61, 69, 77, 85, 93, 101, 109, 117, 125, 133, 141, 149, 157, 165, 173, 181, and 189, or a substantially similar sequence thereof having at least 90%, at least 95%, at least 98% or at least 99% sequence identity. The antibody or antigen-binding fragment may comprise the three light chain CDRs (LCVR1, LCVR2, LCVR3) contained within a light chain variable region (LCVR) having an amino acid sequence selected from the group consisting of SEQ ID NOs:6 and 15, or a substantially similar sequence thereof having at least 90%, at least 95%, at least 98% or at least 99% sequence identity. Alternatively, the antibody or antigen-binding fragment may comprise the three light chain CDRs (LCVR1, LCVR2, LCVR3) contained within a light chain variable region (LCVR) having an amino acid sequence selected from the group consisting of SEQ ID NOs 41, 49, 57, 65, 73, 81, 89, 97, 105, 113, 121, 129, 137, 145, 153, 161, 169, 177, 185, and 193, or a substantially similar sequence thereof having at least 90%, at least 95%, at least 98% or at least 99% sequence identity.

In certain embodiments of the present invention, the antibody or antigen-binding fragment thereof comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs: 1/6 and 11/15. Alternatively, in certain embodiments of the present invention, the antibody or antigen-binding protein comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs:37/41, 45/49, 53/57, 61/65, 69/73, 77/81, 85/89, 93/97, 101/105, 109/113, 117/121, 125/129, 133/137, 141/145, 149/153, 157/161, 165/169, 173/177, 181/185, and 189/193.

In certain embodiments of the present invention, the anti-PCSK9 antibody, or antigen-binding fragment thereof, that can be used in the methods of the present invention has HCDR1/HCDR2/HCDR3/LCDR1/LCDR2/LCDR3 amino acid sequences selected from SEQ ID NOs: 2/3/4/7/8/10 (mAb316P) and 12/13/14/16/17/18 (mAb300N) (See US Patent App. Publ No. 2010/0166768).

In certain embodiments of the present invention, the antibody or antigen-binding fragment thereof comprises HCVR/LCVR amino acid sequence pairs selected from the group consisting of SEQ ID NOs: 1/6 and 11/15. Alternatively, in certain embodiments of the present invention, the antibody or antigen-binding protein comprises the heavy and light chain variable region amino acid sequence pairs (HCVR/LCVR) selected from the group consisting of SEQ ID NOs:37/41, 45/49, 53/57, 61/65, 69/73, 77/81, 85/89, 93/97, 101/105, 109/113, 117/121, 125/129, 133/137, 141/145, 149/153, 157/161, 165/169, 173/177, 181/185, and 189/193.

### Pharmaceutical Compositions and Methods of Administration

The present invention includes methods which comprise administering a PCSK9 inhibitor to a high cardiovascular risk patient, wherein the PCSK9 inhibitor is contained within a pharmaceutical composition. The pharmaceutical compositions of the invention are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN^{™}), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Various delivery systems are known and can be used to administer the pharmaceutical composition of the invention, e.g., encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition of the present invention can be delivered subcutaneously or intravenously with a standard needle and syringe. In addition, with respect to subcutaneous delivery, a pen delivery device readily has applications in delivering a pharmaceutical composition of the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition of the present invention. Examples include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (sanofi-aventis, Frankfurt, Germany), to name only a few. Examples of disposable pen delivery devices having applications in subcutaneous delivery of a pharmaceutical composition of the present invention include, but are not limited to the SOLOSTAR^{™} pen (sanofi-aventis), the FLEXPEN^{™} (Novo Nordisk), and the KWIKPEN^{™} (Eli Lilly), the SURECLICKTM Autoinjector (Amgen, Thousand Oaks, CA), the PENLETTM (Haselmeier, Stuttgart, Germany), the EPIPEN (Dey, L.P.), and the HUMIRATM Pen (Abbott Labs, Abbott Park IL), to name only a few.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, e.g., Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

### Dosage

The amount of PCSK9 inhibitor (e.g., anti-PCSK9 antibody) administered to a subject according to the methods of the present invention is, generally, a therapeutically effective amount. As used herein, the phrase "therapeutically effective amount" means a dose of PCSK9 inhibitor that results in a detectable improvement (at least about 5%, 10%, 15%, 20%, 25%, 30%, 35%, 40%, 45%, 50%, 55%, 60%, 65%, 70%, 75%, or more from baseline) in one or more parameters selected from the group consisting of LDL-C, ApoB100, non-HDL-C, total cholesterol, VLDL-C, triglycerides, Lp(a) and remnant cholesterol.

In the case of an anti-PCSK9 antibody, a therapeutically effective amount can be from about 0.05 mg to about 600 mg, e.g., about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 75 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-PCSK9 antibody. In some embodiments, the therapeutically effective amount of anti-PCSK9 antibody is about 75 mg. In other embodiments, the therapeutically effective amount of anti-PCSK9 antibody is about 150 mg.

The amount of anti-PCSK9 antibody contained within the individual doses may be expressed in terms of milligrams of antibody per kilogram of patient body weight (i.e., mg/kg). For example, the anti-PCSK9 antibody may be administered to a patient at a dose of about 0.0001 to about 10 mg/kg of patient body weight.

### Combination Therapies

As d escribed elsewhere herein, the methods of the present invention may comprise administering a PCSK9 inhibitor to a high cardiovascular risk patient in combination with the patient's previously prescribed a stable daily maximum tolerated dose therapeutic statin regimen. According to certain embodiments of the present invention, additional therapeutic agents, besides a statin, may be administered to a high cardiovascular risk patient in combination with the PCSK9 inhibitor. Examples of such additional therapeutic agents include e.g., (1) an agent which inhibits cholesterol uptake and or bile acid re-absorption (*e.g.*, ezetimibe); (2) an agent which increase lipoprotein catabolism (such as niacin); and/or (3) activators of the LXR transcription factor that plays a role in cholesterol elimination such as 22-hydroxycholesterol.

### Administration Regimens

According to certain embodiments of the present invention, multiple doses of a PCSK9 inhibitor (*i.e*., a pharmaceutical composition comprising a PCSK9 inhibitor) may be administered to a high cardiovascular risk subject over a defined time course (*e.g*., on top of a daily therapeutic statin regimen). The methods according to this aspect of the invention comprise sequentially administering to a subject multiple doses of a PCSK9 inhibitor. As used herein, "sequentially administering" means that each dose of PCSK9 inhibitor is administered to the subject at a different point in time, e.g., on different days separated by a predetermined interval (e.g., hours, days, weeks or months). The present invention includes methods which comprise sequentially administering to the a high cardiovascular risk patient a single initial dose of a PCSK9 inhibitor, followed by one or more secondary doses of the PCSK9 inhibitor, and optionally followed by one or more tertiary doses of the PCSK9 inhibitor.

The terms "initial dose," "secondary doses," and "tertiary doses," refer to the temporal sequence of administration of the individual doses of a pharmaceutical composition comprising a PCSK9 inhibitor. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of the PCSK9 inhibitor, but generally may differ from one another in terms of frequency of administration. In certain embodiments, however, the amount of PCSK9 inhibitor contained in the initial, secondary and/or tertiary doses varies from one another (e.g., adjusted up or down as appropriate) during the course of treatment. In certain embodiments, two or more (e.g., 2, 3, 4, or 5) doses are administered at the beginning of the treatment regimen as "loading doses" followed by subsequent doses that are administered on a less frequent basis (e.g., "maintenance doses").

According to exemplary embodiments of the present invention, each secondary and/or tertiary dose is administered 1 to 26 (e.g., 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, 15, 15½, 16, 16½, 17, 17½, 18, 18½, 19, 19½, 20, 20½, 21, 21½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of antigen-binding molecule which is administered to a patient prior to the administration of the very next dose in the sequence with no intervening doses.

The methods according to this aspect of the invention may comprise administering to a high cardiovascular risk patient any number of secondary and/or tertiary doses of a PCSK9 inhibitor. For example, in certain embodiments, only a single secondary dose is administered to the patient. In other embodiments, two or more (e.g., 2, 3, 4, 5, 6, 7, 8, or more) secondary doses are administered to the patient. Likewise, in certain embodiments, only a single tertiary dose is administered to the patient. In other embodiments, two or more (e.g., 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 2, 4, 6, 8 or more weeks after the immediately preceding dose. Similarly, in embodiments involving multiple tertiary doses, each tertiary dose may be administered at the same frequency as the other tertiary doses. For example, each tertiary dose may be administered to the patient 1 to 2, 4, 6, 8 or more weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

In certain embodiments, the anti-PCSK9 antibody is administered to a subject at a dose of about 75 mg every two weeks, for example for at least two doses.

In certain embodiments, the anti-PCSK9 antibody is administered to a subject at a dose of about 150 mg every two weeks, for example for at least two doses.

The present invention includes administration regimens comprising an up-titration option (also referred to herein as "dose modification"). As used herein, an "up-titration option" means that, after receiving one or more doses of a PCSK9 inhibitor, if a high cardiovascular risk patient has not achieved a specified reduction in one or more defined therapeutic parameters, the dose of the PCSK9 inhibitor is thereafter increased. For example, in the case of a therapeutic regimen comprising administration of 75 mg doses of an anti-PCSK9 antibody to a high cardiovascular risk patient at a frequency of once every two weeks, if after 4 weeks (i.e., 2 doses), the high cardiovascular risk patient has a serum LDL-C concentration ≥50 mg/dL, then the dose of anti-PCSK9 antibody is increased to 150 mg administered once every two weeks thereafter.

In some embodiments, the antibody is administered to a subject at a dose of about 75 mg every two weeks for 4 weeks, and the dose remains at 75 mg every two weeks if, at week 4, the subject's LDL-C value is <50 mg/dL.

In additional embodiments, if the patient's LDL-C is <25 mg/dL on any 2 consecutive measurements on a dose of 150 mg, the dose is thereafter reduced to 75 mg.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human PCSK9

Human anti-PCSK9 antibodies were generated as described in US Patent No. 8,062,640. The exemplary PCSK9 inhibitor used in the following Example is the human anti-PCSK9 antibody designated "mAb316P," also known as "Alirocumab." The mAb316P has the following amino acid sequence characteristics: heavy chain variable region (HCVR) comprising SEQ ID NO:1; light chain variable domain (LCVR) comprising SEQ ID NO:6; heavy chain complementarity determining region 1 (HCDR1) comprising SEQ ID NO:2; HCDR2 comprising SEQ ID NO:3; HCDR3 comprising SEQ ID NO:4; light chain complementarity determining region 1 (LCDR1) comprising SEQ ID NO:7; LCDR2 comprising SEQ ID NO:8; and LCDR3 comprising SEQ ID NO:10.

### Example 2: Effect of Alirocumab, a Monoclonal Antibody to PCSK9, on Long-term Cardiovascular Outcomes Following Acute Coronary Syndrome

### INTRODUCTION

Statins have been approved for clinical use since 1987. Since that time, no lipid-modifying therapy has been proven to improve cardiovascular outcomes on a background of statin treatment. However, the treatments tested to date, including niacin, fenofibrate, ezetimibe, pioglitazone, and dalcetrapib, have modest effects on LDL-C. Inhibition of PCSK9 provides an opportunity to test whether substantial further reduction of LDL-C and other atherogenic lipoproteins can provide further improvement in cardiovascular outcomes beyond those afforded by statins.

This study will determine whether alirocumab, a fully human monoclonal antibody to PCSK9, reduces cardiovascular risk when added to optimal statin therapy. Patients with recent Acute Coronary Syndrome were chosen as the study population because they face a higher risk of recurrent events than patients with stable cardiovascular disease, and therefore might derive a larger absolute benefit from an effective new treatment. The study population was further defined by minimum qualifying levels of atherogenic lipoproteins in order to target those whose residual cardiovascular risk is likely to be modified by further reduction of these lipoproteins. This study will also provide substantial information regarding the safety of PCSK9 inhibition. The study was initiated in 2012 and is ongoing.

### STUDY OBJECTIVE

The present study is an investigator-initiated, international, multicenter, randomized, double-blind, placebo-controlled study in approximately 18,000 patients with recent Acute Coronary Syndrome (ACS). The primary objective is to evaluate whether alirocumab (75-150 mg by subcutaneous injection every 2 weeks), initiated 1-12 months after an index ACS event, reduced the incidence of the composite outcome of coronary heart disease death, major non-fatal coronary events (acute myocardial infarction or hospitalization for unstable angina), or ischemic stroke.

### STUDY POPULATION

Principal inclusion criteria are: 1) hospitalization for ACS, defined by symptoms of myocardial ischemia with an unstable pattern, occurring at rest or with minimal exertion, within 72 hours of an unscheduled hospital admission, due to presumed or proven obstructive coronary disease and at least one of the following: a) elevated cardiac biomarkers, or b) resting ECG changes consistent with ischemia or infarction, plus additional evidence of obstructive coronary disease from regional wall motion or perfusion abnormality, ≥70% epicardial coronary stenosis by angiography, or need for coronary revascularization procedure; and 2) lipid levels inadequately controlled by atorvastatin 40-80 mg or rosuvastatin 20-40 mg daily or maximum tolerated dose of one of these agents, defined by at least one of the following: a) LDL-C ≥70 mg/dl, b) non-HDL-C ≥100 mg/dl, or c) apolipoprotein B ≥80 mg/dl.

Principal exclusion criteria are: 1) age <40 years; 2) qualifying index ACS event <4 or >52 weeks before randomization; 3) not on stable lipid-modifying therapy for at least 2 weeks before randomization; 4) uncontrolled hypertension (>180 mm Hg systolic and/or >110 mm Hg diastolic at randomization visit); 5) New York Heart Association Class III or IV congestive heart failure persisting despite treatment or LVEF <25% if measured; 6) history of hemorrhagic stroke; 7) fasting triglycerides >400 mg/dl (4.52 mmol/L) at qualifying laboratory visit; 8) recurrent ACS event within 2 weeks prior to randomization visit; 9) coronary revascularization procedure performed within 2 weeks prior to randomization visit or planned after randomization; 10) liver transaminases >3 times upper limit of normal; laboratory evidence of current hepatitis B or C infection; creatine kinase >3 times upper limit of normal; estimated glomerular filtration rate <30 ml/min/1.73m2; positive urine or serum pregnancy test; 11) recent diagnosis of hypothyroidism with treatment initiated within 1 month of qualifying laboratory visit; 12) cancer with past 5 years except for adequately treated basal or squamous cell skin cancer or in situ cervical cancer; 13) previous treatment with any PCSK9 antibody; 14) use of fibrates, other than fenofibrate or fenofibric acid, during the run-in period; and 15) inability to provide informed consent or comply with study requirements; pregnancy, lactation, or childbearing potential without use of effective contraception.

The trial is enrolling male and female patients at least 40 years of age who were hospitalized for ACS defined by unstable symptoms of myocardial ischemia occurring at rest or minimal exertion within 72 hours of an unscheduled hospital admission, due to presumed or proven obstructive coronary disease. In addition, a qualifying ACS event requires at least one of the following criteria to be fulfilled: elevated cardiac biomarkers consistent with acute myocardial infarction, or resting ECG changes consistent with ischemia or infarction along with additional evidence of obstructive coronary disease from regional perfusion imaging or wall motion abnormalities, epicardial coronary stenosis ≥70% by angiography, or need for coronary revascularization related to the event.

Qualifying patients must have demonstrated inadequate control of atherogenic lipoproteins despite steady state (at least 2 weeks') treatment with atorvastatin 40-80 mg daily, rosuvastatin 20-40 mg daily, or the maximum tolerated dose of one of these agents. Thus, the background statin treatment in the trial is concordant with secondary prevention guidelines of the American Heart Association and American College of Cardiology for reduction of blood cholesterol levels (see Stone et al. 2013 ACC/AHA Guideline on the Treatment of Blood Cholesterol to Reduce Atherosclerotic Cardiovascular Risk in Adults: A Report of the American College of Cardiology/American Heart Association Task Force on Practice Guidelines. Circulation 2013). Inadequate control of atherogenic lipoproteins was defined by at least one of the following: LDL-C ≥70 mg/dL (1.81 mmol/L), non-high density lipoprotein cholesterol (non-HDL-C) ≥100 mg/dL (2.59 mmol/L), or apolipoprotein B ≥80 mg/dL (0.8 mmol/L).

### STUDY PROCEDURES

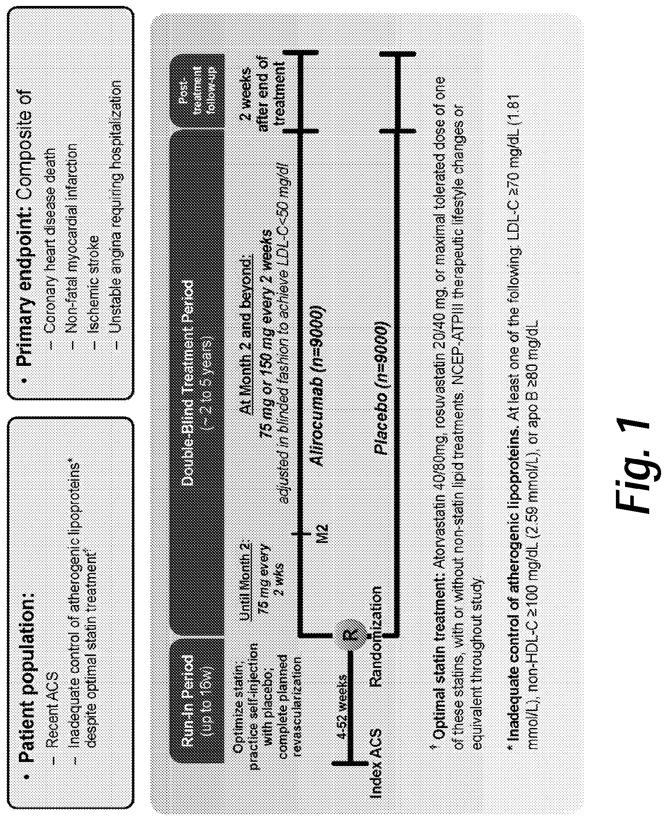

**Figure** 1 schematizes the key phases of the trial. Patients enter a run-in period of duration 2-16 weeks. During this period, patients are instructed in the technique of self-injection using the study auto-injector device. Atorvastatin (40-80 mg daily) or rosuvastatin (20-40 mg daily) is initiated and/or adjusted as necessary to determine the maximum tolerated dose. Other non-excluded lipid-modifying therapies may also be initiated during the run-in period, at the investigator's discretion. After at least two weeks of steady-state lipid modifying therapy, a fasting blood sample is obtained to determine if at least one of the qualifying lipoprotein criteria is met.

Patients who meet all inclusion and no exclusion criteria at the end of the run-in period are randomly assigned to initial treatment with alirocumab 75 mg (1 ml injection volume) subcutaneously every 2 weeks, or matching placebo. Follow-up visits occur 1, 2, 4, 8, 12, 16, 20, and 24 months after randomization, then at 6 month intervals until the common study end date. At randomization and at multiple time points after randomization, patients are assessed for study end points and adverse events, and blood and urine samples are collected for measurements including lipoproteins and apolipoproteins; hematology and chemistry studies including liver, muscle, and kidney function tests; hemoglobin A1c; high sensitivity C-reactive protein (hsCRP), anti-alirocumab antibodies; and pregnancy testing in women of child-bearing potential. LDL-C is calculated using the Friedewald formula, except that values <15 mg/dl were confirmed by direct measurement (as well as when the TG values exceeded 400 mg/dL (4.52 mmol/L)). Samples are also collected for measurement of PCSK9 levels, lipoprotein subfractions, and other mediators of inflammation and cardiovascular risk. An electrocardiogram is recorded at randomization and at study completion. During the randomized treatment period, lipoprotein levels remain blinded to patients and investigators, and treating physicians are instructed to refrain from usual clinical lipoprotein testing.

This study is seeking to determine whether achieving LDL-C levels in the lower portion of the physiologic range improves clinical outcomes; the trial was not designed to explore the safety of sustained, sub-physiologic LDL-C levels. Accordingly, blinded dose adjustment and monitoring procedures are incorporated in the protocol, as follows. Among patients assigned to treatment with alirocumab, if LDL-C measured 1 month after randomization (i.e., after 2 doses of alirocumab 75 mg every 2 weeks) remains ≥50 mg/dl, the dose of alirocumab is increased in a blinded fashion to 150 mg every 2 weeks. If LDL-C measured 1 month after randomization is <50 mg/dl, the dose of alirocumab is maintained at 75 mg. If LDL-C is <25 mg/dl on any 2 consecutive measurements on alirocumab 150 mg, the dose is reduced to 75 mg. If LDL-C is <25 mg/dl but ≥15 mg/dl on 2 consecutive measurements on alirocumab 75 mg, that dose is continued but the patient is monitored for potentially related adverse events by an independent safety physician who reports individual and aggregate findings to the Data Safety Monitoring Board (DSMB) and recommends blinded discontinuation of treatment if data suggest that an adverse event is causally related to treatment. If LDL-C is <15 mg/dl on 2 consecutive measurements during treatment with alirocumab 75 mg, active treatment is discontinued at the next study visit and placebo injections are substituted in a blinded manner for the remaining duration of the study. In composite, these blinded dose adjustments are intended to maximize the number of patients in the alirocumab group with LDL-C<50 mg/dl, while minimizing the number of patients with sustained levels of LDL-C<15 mg/dl.

### STUDY OUTCOMES

The primary efficacy measure is the time to first occurrence of coronary heart disease death, major non-fatal coronary event (myocardial infarction or hospitalization for unstable angina), or ischemic stroke.

Coronary Heart Disease Death is defined as the subset of cardiovascular deaths for which there is a clear relationship to underlying coronary heart disease, including death secondary to acute myocardial infarction (MI), sudden death, heart failure, complication of a coronary revascularization procedure performed for symptoms, coronary disease progression, or new myocardial ischemia where the cause of death is clearly related to the procedure, unobserved and unexpected death, and other death that cannot definitely be attributed to a nonvascular cause.

Acute non-fatal myocardial infarction was defined and sub-classified in accordance with ACC/AHA/ESC Universal Definition of Myocardial Infarction (see Thygesen et al. Third universal definition of myocardial infarction. Eur Heart J 2012;33(20):2551-2567). Silent myocardial infarction is not considered part of the primary end point.

Ischemic stroke is defined as: 1) an acute episode of focal cerebral, spinal, or retinal dysfunction caused by infarction, defined by at least one of the following: a) pathological, imaging, or other objective evidence of acute, focal cerebral, spinal, or retinal ischemic injury in a defined vascular distribution; or b) symptoms of acute cerebral, spinal, or retinal ischemic injury persisting ≥24 hours or until death, with other etiologies excluded; 2) hemorrhagic infarction is considered an ischemic stroke, but stroke caused by intracerebral or subarachnoid hemorrhage is not; or 3) strokes not otherwise sub-classified are considered part of the primary end point.

Hospitalization for unstable angina was defined as: admission to hospital or emergency department with symptoms of myocardial ischemia with an accelerating tempo in the prior 48 hours and/or rest chest discomfort ≥20 min, requiring in addition both of the following: a) new or presumed new ischemic ECG changes, defined by ST depression >0.5 mm in 2 contiguous leads; T-wave inversion >1 mm in 2 contiguous leads with prominent R-wave or R/S>1; ST elevation in >2 contiguous leads >0.2 mV in V2 or V3 in men, >0.15 mV in V2 or V3 in women, or >0.1 mV in other leads; or LBBB; and b) definite contemporary evidence of coronary obstruction by need for coronary revascularization procedure or at least one epicardial stenosis ≥70%. Procedures or stenoses due only to restenosis at prior PCI site are excluded.

Secondary end points include ischemia-driven coronary revascularization procedures, hospitalization for congestive heart failure, and all-cause mortality.

The primary efficacy measures are: time to first occurrence of coronary heart disease death, non-fatal acute myocardial infarction, fatal or non-fatal ischemic stroke, or unstable angina requiring hospitalization.

The main secondary efficacy measures are (in hierarchical order): 1) time from randomization to first occurrence of major coronary heart disease event (coronary heart disease death or non-fatal myocardial infarction), unstable angina requiring hospitalization, or ischemia-driven coronary revascularization procedure (PCI or CABG, excluding procedures performed solely for restenosis at prior PCI site). Ischemia-driven coronary revascularization must be driven by one of the following: a) acute ischemia (ACS), or b) new or progressive symptoms (angina or equivalent) or new or progressive functional testing abnormalities (e.g., stress testing or imaging); 2) time from randomization to first occurrence of a major coronary heart disease event; 3) time from randomization to first occurrence of any cardiovascular event (any cardiovascular death, any non-fatal coronary heart disease event, or non-fatal ischemic stroke); 4) time from randomization to first occurrence of all-cause mortality, non-fatal myocardial infarction, or non-fatal ischemic stroke; and 5) time from randomization to death (all-cause mortality).

Other secondary efficacy measures are: 1) time from randomization to coronary heart disease death; 2) time from randomization to first occurrence of non-fatal myocardial infarction; 3) time from randomization to first occurrence of ischemic stroke; 4) time from randomization to first occurrence of unstable angina requiring hospitalization; 5) time from randomization to first occurrence of ischemia-driven coronary revascularization procedure; and 6) time from randomization to first occurrence of congestive heart failure requiring hospitalization.

Safety measures are: all adverse events, heart rate and blood pressure, hematology and biochemistry assessments.

Other measures are: 1) anti-alirocumab antibodies assessed throughout the study; and 2) percent change in LDL cholesterol, apolipoprotein B, non-HDL cholesterol, and high sensitivity C-reactive protein.

Laboratory efficacy end points include change from baseline in calculated LDL-C, apolipoprotein B, non-HDL-C, and hsCRP. Safety of alirocumab treatment is assessed by reporting of adverse events and laboratory tests. Adverse events of special interest in this trial include allergic events, local injection site reactions, liver enzyme increase, and hemolytic anemia.

### STATISTICAL CONSIDERATIONS

The projected Kaplan-Meier incidence of a primary end point event in the placebo group is 3.8% at 12 months, 6.4% at 24 months, 9.0% at 36 months, and 11.4% at 48 months. Other assumptions include 1% of patients lost to follow-up through 24 months, a median LDL-C at baseline of 90 mg/dl, and a 50% reduction of LDL-C from baseline with alirocumab treatment, resulting in a 15% hazard reduction. Based on these assumptions and specifying a log-rank test at a 1-sided 2.49% significance level to account for two interim analyses, the trial will have 90% power with 1613 primary end point events, corresponding to a sample size of 18,000 patients randomized over 40 months. To allow sufficient duration of exposure to alirocumab for thorough assessments of safety and efficacy, the trial will continue until 1613 primary end point events have occurred and all evaluable surviving patients have been followed for at least 2 years. For the primary outcome, treatment effects will be examined across subgroups categorized according to gender, age, race, geographical region, and time from ACS event to randomization. Time-to-event secondary outcomes will be analyzed using the same methodology as for the primary end point. For main secondary outcomes, the overall Type 1 error will be controlled by use of a sequential inferential approach. Proportional hazards regression models will be constructed to include changes in or absolute values of LDL-C and other lipid parameters. Analysis of subpopulations defined by categorical and continuous variables will be performed according to a pre-specified statistical analysis plan. Safety results will be presented by treatment group without formal inferential testing.

The independent DSMB, composed of 3 cardiologists, 1 lipidologist, and 1 statistician, review interim data at regular intervals to assess safety and efficacy. When approximately 50% of events have occurred, the DSMB will conduct an interim analysis for futility (non-binding boundary corresponding to hazard ratio >1.008). When approximately 75% of events have occurred, the DSMB will conduct a second interim analysis for futility (non-binding boundary corresponding to hazard ratio >0.951) and overwhelming efficacy (hazard ratio <0.802 corresponding to p<0.0001 for the primary end point with consistency across subgroups and regions, positive trends for secondary end points including all-cause mortality, and no excess non-cardiovascular mortality).

### Example 3: Long-term safety and tolerability of Alirocumab in high cardiovascular risk patients with hypercholesterolemia not adequately controlled with their lipid modifying therapy: a randomized, double-blind, placebo-controlled study

### INTRODUCTION

This study was undertaken to assess the long-term safety and tolerability of alirocumab in patients at high cardiovascular risk who are not at LDL-C goal. This population that is not at LDL-C goal on optimized LMT represents a highest risk group with a well identified unmet medical need that can be addressed by adding alirocumab to their LDL-C modifying therapies. Two sets of results are reported: (1) a pre-specified interim analysis was performed when all patients reached one year and approximately 25 percent of patients reached 18 months of treatment; and (2) the final analysis of the safety population, when all patients had completed the study.

### STUDY OBJECTIVES

The primary study objective was to evaluate the long-term safety and tolerability of alirocumab in high cardiovascular risk patients with hypercholesterolemia not adequately controlled with their lipid modifying therapy.

Secondary study objectives included evaluation of the effect of alirocumab on various lipid components associated with hypercholesterolemia, including for example levels of low-density lipoprotein cholesterol (LDL-C), Apolipoprotein B (Apo B), non-high-density lipoprotein cholesterol (non-HDL-C), total cholesterol (total-C), lipoprotein a (Lp [a]), high-density lipoprotein cholesterol (HDL-C), triglyceride (TG), and apolipoprotein A-1 (Apo A-1).

This study also evaluated cardiovascular effects.

### STUDY DESIGN

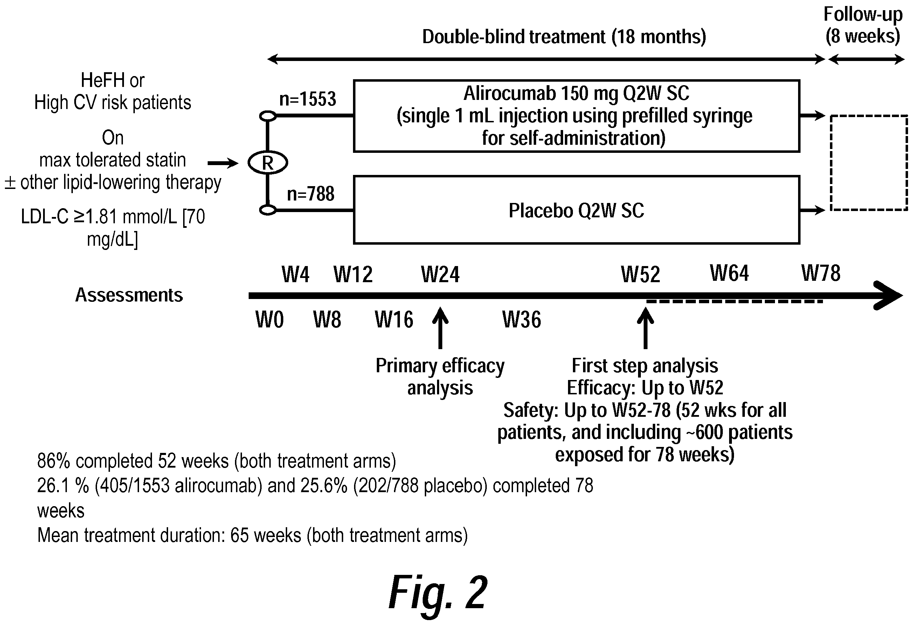

This was a randomized, double-blind, placebo-controlled, unbalanced (2:1, alirocumab:placebo), parallel-group, multi-center, multi-national study evaluating the long-term safety and tolerability of alirocumab in high cardiovascular risk patients with hypercholesterolemia who were not adequately controlled with a maximally tolerated daily registered dose of a statin with or without other lipid modifying therapy. See **Figure 2****.** Patients were stratified according to heFH population, prior history of MI or ischemic stroke, statin treatment and geographic region. Patients at high cardiovascular risk were defined as 1) having heFH (who may or may not have CHD/CHD risk equivalents) or 2) no prior diagnosis of heFH but having hypercholesterolemia together with established CHD or CHD risk equivalents. Patients must have been hypercholesterolemic and not adequately controlled (i.e., LDL-C ≥70 mg/dL [≥1.81 mmol/L]) despite therapy with maximally tolerated daily registered dose of a statin with or without other lipid modifying therapy at a stable dose for at least 4 weeks (6 weeks for fenofibrate) prior to screening.

### Description of the protocol

Patients randomized to alirocumab received 150 mg every 2 weeks. The study consisted of 3 periods: screening, double-blind treatment, and follow up. The screening period was up to 3 weeks in duration. The double-blind treatment period was a randomized, double-blind study treatment period of 18 months. The follow-up period was a period of 8 weeks after the end of the double-blind treatment period.

### SELECTION OF PATIENTS

This study was designed to randomize approximately 2100 patients in a 2:1 randomization ratio to the following: Alirocumab: approximately 1400 patients; placebo: approximately 700 patients.

Patients meeting the following criteria were considered for enrollment into the study: 1) patients with heterozygous Familial Hypercholesterolemia (heFH)* with or without established coronary heart disease (CHD) or CHD risk equivalents who were not adequately controlled with a maximally tolerated stable daily dose of statin** for at least 4 weeks prior to the screening visit (Week -3) with or without other lipid modifying therapy (LMT); or 2) patients with hypercholesterolemia and established CHD or CHD risk equivalents (see herein/below for definitions) who were not adequately controlled with a maximally tolerated stable daily dose of statin** for at least 4 weeks prior to the screening visit (Week -3) with or without other lipid modifying therapy (LMT).

*Diagnosis of heFH must have been made either by genotyping or by clinical criteria. For those patients not genotyped, the clinical diagnosis may have been based on either the WHO criteria/Dutch Lipid Clinical Network criteria with a score > 8 points or the Simon Broome register diagnostic criteria with a criterion for definite FH.

** Definition of maximally tolerated dose (any of the following were acceptable): 1) rosuvastatin 20 mg or 40 mg daily; 2) atorvastatin 40 mg or 80 mg daily; 3) simvastatin 80 mg daily (if already on this dose for >1 year); 4) patients not able to be on any of the above statin doses, should have been treated with the dose of daily atorvastatin, rosuvastatin or simvastatin which was considered appropriate for the patient as per the investigator's judgment or concerns. Some examples of acceptable reasons for a patient taking a lower statin dose included, but were not limited to: adverse effects on higher doses, advanced age, low body mass index, regional practices, local prescribing information, concomitant medications, co-morbid conditions such as impaired glucose tolerance/impaired fasting glucose.

Documented history of CHD included one or more of the following: i) acute myocardial infarction (MI); ii) silent myocardial infarction; iii) unstable angina; iv) coronary revascularization procedure (eg, percutaneous coronary intervention [PCI] or coronary artery bypass graft surgery [CABG]); and/or v) clinically significant CHD diagnosed by invasive or non-invasive testing (such as coronary angiography, stress test using treadmill, stress echocardiography or nuclear imaging).

CHD risk equivalents included one or more of the following 4 criteria: i) Documented peripheral arterial disease (one of the following criteria [a, b, or c] must be satisfied): current intermittent claudication (muscle discomfort in the lower limb that is both reproducible and produced by exercise and relieved by rest within 10 minutes) of presumed atherosclerotic origin together with ankle-brachial index < 0.90 in either leg at rest, or b) history of intermittent claudication (muscle discomfort in the lower limb that is both reproducible and produced by exercise and relieved by rest within 10 minutes) together with endovascular procedure or surgical intervention in one or both legs because of atherosclerotic disease or c) history of critical limb ischemia together with thrombolysis, endovascular procedure or surgical intervention in one or both legs because of atherosclerotic disease; ii) Documented previous ischemic stroke with a focal ischemic neurological deficit that persisted more than 24 hours, considered as being of atherothrombotic origin. CT or MRI must have been performed to rule out hemorrhage and non-ischemic neurological disease; iii) Documented moderate chronic kidney disease (CKD) as defined by 30 ≤ eGFR <60 mL/min/1.73 m2 for 3 months or more, including the screening visit; iv) Known history of diabetes mellitus AND 2 or more additional risk factors (as listed below): a History of hypertension (established on antihypertensive medication), b Documented history of ankle-brachial index ≤0.90, c Documented history of microalbuminuria or macroalbuminuria or dipstick urinalysis at screening visit (Week-3) with >2+ protein, d Documented history of pre-proliferative or proliferative retinopathy or laser treatment for retinopathy, e Known family history of premature CHD (CHD in father or brother before 55 years of age; CHD in mother or sister before 65 years of age).

According to the Simon Broome Register Diagnostic Criteria for Heterozygous Familial Hypercholesterolemia, definite familial hypercholesterolemia was defined as: Total-C >6.7 mmol/l (260 mg/dL) or LDL cholesterol above 4.0 mmol/l (155 mg/dL) in a child <16 years or Total-C >7.5 mmol/l (290 mg/dL) or LDL cholesterol above 4.9 mmol/l (190 mg/dL) in an adult. (Levels either pre-treatment or highest on treatment) plus Tendon xanthomas in patient, or in 1st degree relative (parent, sibling, child), or in 2nd degree relative (grandparent, uncle, aunt) or DNA-based evidence of an LDL receptor mutation or familial defective apo B-100.

**Table 1**

| **Diagnostic Scoring for Heterozygous Familial Hypercholesterolemia** | | | | |
|---|---|---|---|---|
| Family history | | | | |
| a | First degree relative with known premature (men <55 yrs, women <60 yrs) coronary and vascular disease. | | | 1 |
| b | First degree relative with known LDL-cholesterol >95th percentile for age and sex. | | | |
| and/or | | | | |
| a | First degree relative with tendon xanthomata and/or arcus cornealis. | | | 2 |
| b | Children below 18 yrs. with LDL-cholesterol >95th percentile for age and sex. | | | |
| | | | | |

| Clinical history | | | | |
|---|---|---|---|---|
| a | Patient has premature (men <55 yrs, women <60 yrs) coronary artery disease | | | 2 |
| b | Patient has premature (men <55 yrs, women <60 yrs) cerebral or peripheral vascular disease. | | | 1 |
| | | | | |

| Physical examination | | | | |
|---|---|---|---|---|
| a | Tendon xanthomata | | | 6 |
| b | Arcus corneal is below the age of 45 yrs. | | | 4 |
| | | | | |

| Laboratory analysis | | | | |
|---|---|---|---|---|
| | | mmol/L | mg/dL | |
| a | LDL-cholesterol | >8.5 | >330 | 8 |
| b | LDL-cholesterol | 6.5-8.4 | 250-329 | 5 |
| c | LDL-cholesterol | 5.0-6.4 | 190-249 | 3 |
| d | LDL-cholesterol | 4.0-4.9 | 155-189 | 1 |
| (HDL-cholesterol and triglycerides are normal) | | | | |
| | | | | |

| DNA-analysis | | | | |
|---|---|---|---|---|
| a | Functional mutation low-density lipoprotein receptor gene present | | | 8 |
| | | | | |

| Diagnosis of heFH is: | | | | |
|---|---|---|---|---|
| Certain When | | | >8 points | |
| Probable When | | | 6-8 points | |
| Possible When | | | 3-5 points | |

According to the Simon Broome Register Diagnostic Criteria for Heterozygous Familial Hypercholesterolemia, possible familial hypercholesterolemia was defined as: Total-C >6.7 mmol/l (260 mg/dL) or LDL cholesterol above 4.0 mmol/l (155 mg/dL) in a child <16 years or Total-C >7.5 mmol/l (290 mg/dL) or LDL cholesterol above 4.9 mmol/l (190 mg/dL) in an adult. (Levels either pre-treatment or highest on treatment) and at least one of the following: family history of myocardial infarction below 50 years of age in 2nd degree relative or below 60 years of age in 1st degree relative, or family history of raised cholesterols >7.5 mmol/l (290 mg/dL) in adult 1st or 2nd degree relative or >6.7 mmol/l (260 mg/dL) in child or sibling under 16 years of age.

The WHO Criteria (Dutch Lipid Network clinical criteria) for diagnosis of Heterozygous Familial Hypercholesterolemia (heFH) is set forth in Table 1.

Patients who met all the above inclusion criteria were screened for the following exclusion criteria.

Exclusion criteria related to study methodology were: 1) Without established history of CHD or CHD risk equivalents or without a diagnosis of heFH based on genotyping or clinical criteria; 2) LDL-C <70 mg/dL (<1.81 mmol/L) at the screening visit (Week-3); 3) Not on a stable dose of LMT (including statin) for at least 4 weeks and/or fenofibrate for at least 6 weeks, as applicable, prior to the screening visit (Week -3) and from screening to randomization; 4) Currently taking a statin that is not simvastatin, atorvastatin, or rosuvastatin; 5) Simvastatin, atorvastatin, or rosuvastatin is not taken daily or not taken at a registered dose; 6) Daily doses above atorvastatin 80 mg, rosuvastatin 40 mg or simvastatin 40 mg (except for patients on simvastatin 80 mg for more than one year, who are eligible); 7) Use of fibrates other than fenofibrate within 6 weeks prior to screening visit (Week -3) or between screening and randomization visits; 8) Use of nutraceutical products or over-the-counter therapies that may affect lipids which have not been at a stable dose for at least 4 weeks prior to the screening visit (Week -3) or between screening and randomization visits; 9) Use of red yeast rice products within 4 weeks of the screening visit (Week-3) or between screening and randomization visits; 10) Patient who has received plasmapheresis treatment within 2 months prior to the screening visit (Week -3), or has plans to receive it; 11) Recent (within 3 months prior to the screening visit [Week -3] or between screening and randomization visits) MI, unstable angina leading to hospitalization, uncontrolled cardiac arrhythmia, CABG, PCI, carotid surgery or stenting, cerebrovascular accident, transient ischemic attack (TIA), endovascular procedure or surgical intervention for peripheral vascular disease; 12) Planned to undergo scheduled PCI, CABG, carotid or peripheral revascularization during the study; 13) History of New York Heart Association (NYHA) Class III or IV heart failure within the past 12 months; 14) Systolic blood pressure >180 mmHg or diastolic blood pressure >110 mmHg at screening visit or randomization visit; 15) Known history of hemorrhagic stroke; 16) Age < 18 years or legal age of majority at the screening visit (Week-3), whichever is greater; 17) Known history of active optic nerve disease; 18) Patients not previously instructed on a cholesterol-lowering diet prior to the screening visit (Week-3); 19) Known history of homozygous FH; 20) Known history of loss of function of PCSK9 (i.e., genetic mutation or sequence variation); 21) Use of systemic corticosteroids, unless used as replacement therapy for pituitary/adrenal disease with a stable regimen for at least 6 weeks prior to randomization. Note: topical, intra-articular, nasal, inhaled and ophthalmic steroid therapies are not considered as "systemic" and are allowed; 22) Use of continuous hormone replacement therapy unless the regimen has been stable in the past 6 weeks prior to the Screening visit (Week-3) and no plans to change the regimen during the study; 23) History of cancer within the past 5 years, except for adequately treated basal cell skin cancer, squamous cell skin cancer, or in situ cervical cancer; 24) Known history of HIV positivity; 25) Conditions/situations such as: a) Any clinically significant abnormality identified at the time of screening that in the judgment of the Investigator or any sub-investigator would preclude safe completion of the study or constrain endpoints assessment such as major systemic diseases, patients with short life expectancy, b) Patients considered by the Investigator or any sub-investigator as inappropriate for this study for any reason, eg.: i) Those deemed unable to meet specific protocol requirements, such as scheduled visits; ii) Those deemed unable to administer or tolerate long-term injections as per the patient or the investigator; iii) Investigator or any sub-investigator, pharmacist, study coordinator, other study staff or relative thereof directly involved in the conduct of the protocol, etc; iv) Presence of any other conditions (eg, geographic, social....) actual or anticipated, that the Investigator feels would restrict or limit the patient's participation for the duration of the study; 26) Patient who has been previously treated with at least one dose of alirocumab or any other anti-PCSK9 monoclonal antibody in other clinical trials; 27) Patient who has taken any investigational drugs other than alirocumab training placebo kits within 1 month or 5 half lives, whichever is longer; 28) Patient who withdraws consent during the screening period (patient who is not willing to continue or fails to return); 29) Laboratory findings during the screening period (not including randomization labs): A) Positive test for Hepatitis B surface antigen and/or Hepatitis C antibody (confirmed by reflexive testing), B) Triglycerides (TG) >400 mg/dL (>4.52 mmol/L) (1 repeat lab is allowed); C) Positive serum or urine pregnancy test in women of childbearing potential; D) eGFR <30 mL/min/1.73 m2 according to 4-variable MDRD equation; E) HbA1c >10%; F) ALT or AST >3 x ULN (1 repeat lab is allowed); G) CPK >3 x ULN (1 repeat lab is allowed).

Exclusion criteria related to the active comparator and/or mandatory background therapies were: 30) all contraindications to the background therapies or warning/precaution of use (when appropriate) as displayed in the respective National Product Labeling.

Exclusion criteria related to the current knowledge of alirocumab were: 31) Known hypersensitivity to monoclonal antibody therapeutics; 32) Pregnant or breast-feeding women; 33) Women of childbearing potential not protected by highly-effective method(s) of birth control and/or who are unwilling or unable to be tested for pregnancy. Note: Women of childbearing potential must have had a confirmed negative serum pregnancy test at screening and urine pregnancy test at randomization visit. They must have used effective contraceptive methods throughout the study and agreed to repeat urine pregnancy test at designated visits. The applied methods of contraception had to meet the criteria for a highly effective method of birth control according to the "Note for guidance on non-clinical safety studies for the conduct of human clinical trials for pharmaceuticals (CPMP/ICH/286/95)". Postmenopausal women must have been amenorrheic for at least 12 months.

### STUDY TREATMENTS

Sterile alirocumab drug product (IMP) was supplied at a concentration of 150 mg/mL as 1 mL volume. During the double-blind treatment period, alirocumab or placebo was administered subcutaneously as 1 mL injection every 2 weeks, starting at Week 0 continuing up to the last injection (ie, Week 76), which was at 2 weeks before the end of the double blind treatment period.

The following classes of drugs were identified as non-investigational medicinal products (NIMP) because the medication was either a background therapy or a potential rescue medication: statins (rosuvastatin, atorvastatin, simvastatin); cholesterol absorption inhibitors (ezetimibe); bile acid-binding sequestrants (such as cholestyramine, colestipol, colesevelam); nicotinic acid; fenofibrate; omega-3 fatty acids (≥ 1000 mg daily).

Patients were randomized to receive either placebo or alirocumab during the double-blind study treatment period. The randomization ratio alirocumab:placebo was 2:1. The randomization was stratified by heFH population (Yes, No), prior history of acute or silent MI or ischemic stroke (Yes, No), statin treatment (atorvastatin 40 to 80 mg daily or rosuvastatin 20 to 40 mg daily, vs. simvastatin whatever the daily dose, atorvastatin below 40 mg daily or rosuvastatin below 20 mg daily) and region (North America, Western Europe, Eastern Europe and Rest of World).

A concomitant medication was any treatment received by the patient concomitantly to the study (until follow-up visit). Concomitant medications should have been kept to a minimum during the study. However, if these were considered necessary for the patient's welfare and were unlikely to interfere with the IMP, they may have been given at the discretion of the Investigator, with a stable dose (when possible). Besides the specific information related to concomitant medications provided in this section, any other concomitant medication(s) would be allowed and would have to be recorded. If the patient had an LDL-C ≥160 mg/dL (4.14 mmol/L) at the screening visit (Week -3) and was treated with a statin only, i.e., without additional LMT, the investigator would have to report the reason for the patient not being on a second LMT. Nutraceutical products or over-the-counter therapies that may affect lipids were allowed only if they have been used at a stable dose for at least 4 weeks prior to screening visit, during the screening period and maintained during the first 24 weeks of the double-blind treatment period. After the Week 24 visit, modification to these nutraceutical products or over-the-counter therapies was allowed but in general should have been avoided. Examples of such nutraceutical products or over-the-counter therapies included omega-3 fatty acids at doses <1000 mg, plant stanols such as found in Benecol, flax seed oil, and psyllium.

Patients were on maximally tolerated daily registered doses of statins with or without other lipid modifying therapy during the study. From the screening visit (Week -3) until the first 24 weeks of the double-blind treatment period (Week 24), the background lipid modifying therapy should not have been changed. No dose adjustment, discontinuation or initiation of other statins or other lipid modifying therapy should have taken place during this time, barring exceptional circumstances whereby overriding concerns (including but not limited to triglyceride alert posted by the central lab) warranted such changes, as per the investigator's judgment.

The following therapies were not allowed during the study (including the screening period until the follow-up visit): fibrates other than fenofibrate; red yeast rice products; and statin other than simvastatin, atorvastatin, or rosuvastatin.

### SAFETY ASSESSMENT

Safety was assessed by the following parameters: recording of adverse events (including adjudicated cardiovascular events); standard laboratory tests (hematology, chemistry and urinalysis); liver panel (ALT, AST, Alkaline Phosphatase [ ALP], and total bilirubin); creatine phospho kinase (CPK); hepatitis C Antibody (if positive, then confirmed with reflexive testing); vitamin E (alpha-tocopherol) and other fat soluble vitamins; cortisol (with reflexive ATCH levels, as needed, and followed by ACTH stimulation test, as needed); gonadal hormone assessments; electrocardiogram (ECG); vital signs (systolic and diastolic blood pressure and heart rate); physical examination (including neurological exam); color vision test (as a screening test for more comprehensive ophthalmological testing, as needed). Safety parameters (adverse events [including adjudicated cardiovascular events], laboratory data, vital signs, and ECG) were assessed throughout the study.

Safety endpoints assessed in this trial were: cardiovascular events; allergic events; local tolerability at injection site; other adverse events (including hemolytic anemia); laboratory tests: urinalysis, hematology (red blood cell count, red blood cell distribution width (RDW), reticulocyte count, hemoglobin, hematocrit, platelets, white blood cell count with differential blood count), standard chemistry (sodium, potassium, chloride, bicarbonate, calcium, phosphorous, urea nitrogen, creatinine, uric acid, total protein, albumin, LDH, γ Glutamyl Transferase [γGT]), Vitamin E (alpha tocopherol) and other Fat Soluble Vitamins, cortisol (and reflexive ACTH levels, as needed, followed by ACTH stimulation test, as needed), Gonadal Hormone Assessments, Hepatitis C antibody, liver panel (ALT, AST, ALP, and total bilirubin), and CPK; vital signs including heart rate and blood pressure; and 12-lead ECG.

### RESULTS I- PRE-SPECIFIED ANALYSIS

### Summary population characteristics:

A pre-specified interim analysis was performed when all patients reached one year and approximately 25 percent of patients reached 18 months of treatment. A total of 2341 patients were randomized in the study (1553 in alirocumab versus 788 in placebo group). The baseline characteristics of the patient population in this pre-specified analysis are set forth in Table 2. 17.7% of patients had heFH with or without established CHD or CHD risk equivalents. 82.3% of patients were enrolled with non-familial hypercholesterolemia with established CHD or CHD risk equivalents. A large majority of the randomized population (90.6%) had a history of Coronary Heart Disease (CHD) or CHD risk equivalents. CHD was reported for 68.6% of patients (see Table 2).

Demographics characteristics, disease characteristics and lipid parameters at baseline were similar in the alirocumab group as compared to the placebo group. Mean (SD) baseline calculated LDL-C was 122.4 (42.2) mg/dL (3.171 (1.092) mmol/L). With regards to background lipid modifying therapy, 1032 (44.1%) patients took high intensity statin at randomization (i.e. atorvastatin 40 to 80 mg or rosuvastatin 20 to 40 mg daily) and 334 (14.3%) were receiving ezetimibe in addition to the statin(see Table 2).

**Table 2 Baseline Characteristics**

| **Characteristic** | **Alirocumab (n=1553)** | **Placebo (n=788)** |
|---|---|---|
| Age, years, mean (SD) | 60.4 (*10.4*) | 60.6 (*10.4*) |
| Male | 63.3% (983) | 60.2% (474) |
| Race, White | 92.8% (1441) | 92.6% (730) |
| BMI, kg/m², mean (SD) | 30.2 (*5.7*) | 30.5 (*5.5*) |
| HeFH | 17.8% (276) | 17.6% (139) |
| CHD history | 67.9% (1055) | 70.1% (552) |
| CHD risk equivalent^{†} | 41.1% (639) | 41.2% (325) |
| Type 2 diabetes | 34.9% (542) | 33.9% (267) |
| Any statin^{†}, % (n) | 99.9% (1552) | 99.9% (787) |
| High intensity statin^{‡} | 44.4% (690) | 43.4% (342) |
| Any LLT other than statins, % (n) | 28.1% (437) | 27.9% (220) |
| Ezetimibe, % (n) | 13.9% (216) | 15.0% (118) |
| LDL-C (calculated) | 3.2 (*1.1*) | 3.2 (*1.1*) |
| Mean (SD), mmol/L [mg/dL] | 122.7 (*42.6*) | 121.9 (*41.4*) |
| Non-HDL-C, mean (SD), mmol/L [mg/dL] | 4.0 (1.2) | 3.9 (1.2) |
| | [152.6 (46.6)] | [152.0 (45.8)] |
| Apo B, mean (SD), mg/dL | 101.9 (27.7) | 101.4 (27.3) |
| Lp(a), mg/dL, median (IQR) | 22.2 (7.6:66.5) | 20.9 (6.5:66.8) |

| | | |
|---|---|---|
| % (n) of patients unless stated. All patients on background of max tolerated statin±other lipid-lowering therapy. ^{†}Patients should receive either rosuvastatin 20-40 mg, atorvastatin 40-80 mg daily, or simvastatin 80 mg daily unless not tolerated and/or appropriate other dose given according to the judgement of the investigator. ^{‡}High-intensity statin: atorvastatin 40-80 mg or rosuvastatin 20-40 mg daily. | | |

Exposure to injections was similar across treatment groups with a median exposure of 68 weeks. At the cut-off date of the pre-specified analysis, 607 (25.9%) patients have completed the 18-months double-blind treatment period (ie. at least 76 weeks of exposure and the Week 78 visit completed), including at least 400 patients in the alirocumab group, as agreed with Health Authorities during End of Phase 2 meeting consultation: 405 patients (26.1%) in the alirocumab group and 202 patients (25.6%) in the placebo group.

Study disposition, exposure and safety analyses were assessed using all data up to the study common cut-off date and therefore include data beyond week 52 and up to week 78 or up to the follow-up visit. Final results for primary efficacy endpoint (at Week 24) and key secondary efficacy endpoints (assessed up to Week 52) are provided in this first step analysis.

There were in total 525 (22.4%) randomized patients who completed the study treatment period up to the cut-off date, i.e. last IMP injection was taken (Week 76) and end of treatment visit (Week 78) was performed within 21 days after the last IMP injection and at least 525 days after randomization.

In the alirocumab group, 1550 of the 1553 actually received alirocumab. 23% (n=349) of these patients completed 78 weeks, 20% (n=311) discontinued treatment and 57% (n=890) are still receiving treatment. The ITT safety populations for the alirocumab group were 1530 and 1550 patients, respectively. In the placebo group, all 788 received placebo. 22% (n=176) of these patients completed 78 weeks, 19% (n=146) discontinued treatment and 59% (n=466) are still receiving treatment. The ITT safety populations for the alirocumab group were 780 and 788 patients, respectively.

### Primary efficacy endpoint

The intention-to-treat (ITT) analysis includes all LDL-C values collected on-treatment and off-treatment up to Week 52. The primary endpoint (percent change in calculated LDL-C from baseline to Week 24) analysis is provided based on a MMRM model on the ITT population, using LS means estimates at Week 24. 146 (9.5%) patients in the alirocumab group and 72 (9.2%) patients in the placebo group did not have a calculated LDL-C value at Week 24. These missing values were accounted for by the MMRM model.

A statistically significant decrease in percent change in LDL-C from baseline to Week 24 was observed in alirocumab group (N=1530; LS mean versus baseline -61.0%) compared to the placebo group (N=780; LS mean versus baseline 0.8%) (LS mean difference vs. placebo of -61.9%, p<0.0001).

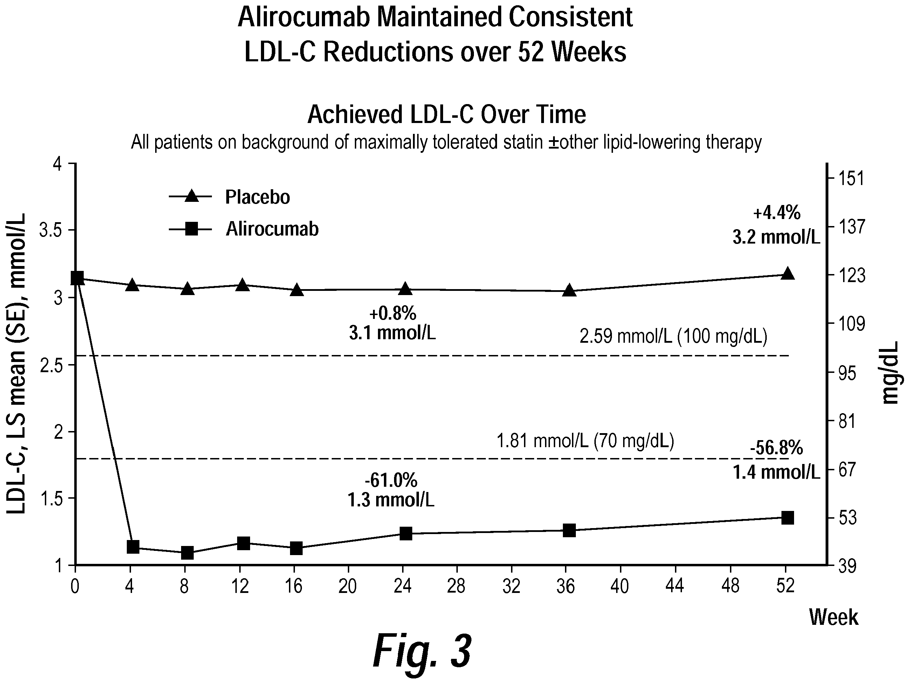

In the alirocumab group, a consistent LDL-C reduction from baseline was observed from week 4 to Week 52 (see Figure 3). The proportion of very high caradiovascular risk patients who reached a calculated LDL-C of less than 70 mg/dL or high cardiovascular risk patients who reached a calculated LDL-C of less than 100 mg/dL was 80.7% in the Alirocumab arm and 8.5% in the placebo arm (p<0.001). The proportion of patients who reached a calculated LDL-C of less than 70 mg/dL, regardless of risk, was 79.3% in the Alirocumab arm and 8.0% in the placebo arm (p<0.001).

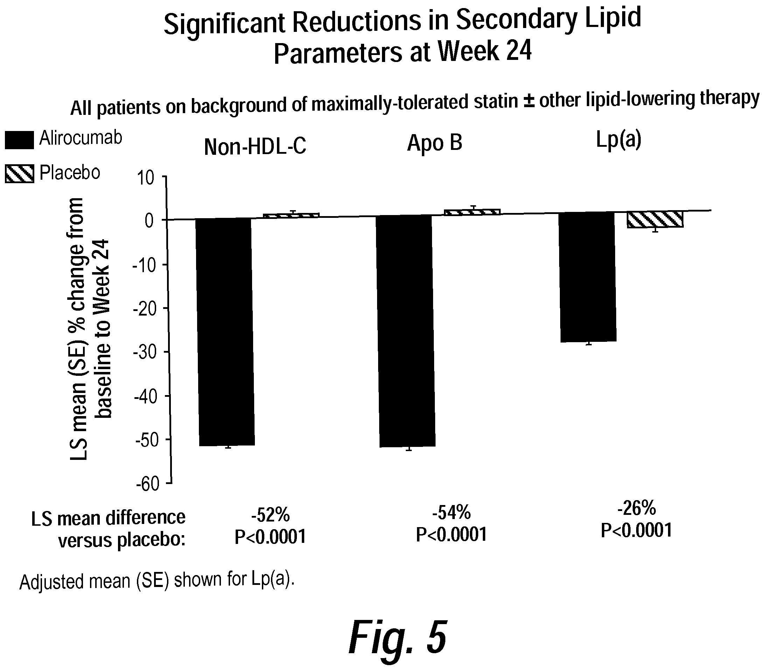

The alirocumab group also exhibited a significant reduction in non-HDL-C, ApoB and Lp(a) levels relative to placebo at 24 weeks (see Figure 5).

### Summary safety results:

At the time of the pre-specified analysis, the percentages of patients who experienced treatment emergent adverse events (TEAE), serious TEAE and TEAE leading to treatment discontinuation were similar between treatment groups.

The most frequently reported SOC in both treatment groups (≥ 10%) were "infections and infestations" (45.5% in the alirocumab group vs. 46.1% in the placebo group), "musculoskeletal and connective tissue disorders" (27.2% in the alirocumab group vs. 28.6% in the placebo group), "gastrointestinal disorders" (18.6% in the alirocumab group vs. 18.8% in the placebo group), "nervous system disorders" (17.0% in the alirocumab group vs. 17.8% in the placebo group), "general disorders and administration site conditions" (15.4% in the alirocumab group vs. 17% in the placebo group), "injury, poisoning and procedural complications" (13.4% in the alirocumab group vs. 14.2% in the placebo group), and "respiratory, thoracic and mediastinal disorders" (11.0% in the alirocumab group vs. 10.9% in the placebo group).

No marked imbalance was observed on the frequency of TEAE (Table 3). The following TEAEs were the most frequently reported in any treatment group (≥ 5% in any group): nasopharyngitis (12.6% in alirocumab vs. 12.7% in placebo group), upper respiratory tract infection (7.0% vs. 8.0%), injection site reaction (5.7% vs 4.3%), influenza (5.4% vs 5.5%), diarrhea (5.3% vs 5.1%), urinary tract infection (5.2% vs. 6.2%), bronchitis (5.2% vs 4.7%) and headache (4.8% vs. 5.6%).

**Table 3 - Overview of adverse event profile: Treatment emergent adverse events - Safety population at the time of the pre-specified analysis)**

| **n(%)** | **Placebo (N=788)** | **Alirocumab 150 Q2W (N=1550)** |
|---|---|---|
| Patients with any TEAE | 635 (80.6%) | 1218 (78.6%) |
| Patients with any serious treatment emergent | 139 (17.6%) | 255 (16.5%) |
| Patients with any TEAE leading to death | 8 (1.0%) | 7 (0.5%) |
| Patients with any TEAE leading to permanent treatment discontinuation | 43 (5.5%) | 96 (6.2%) |

| | | |
|---|---|---|
| n (%) = number and percentage of patients with at least one TEAE | | |

Nineteen (19) deaths were reported during the study (11 (0.7%) in the alirocumab group versus 8 (1.0%) in the placebo group). Focusing on the TEAE leading to death, 7 (0.5%) deaths were reported in the alirocumab group versus 8 (1.0%) in the placebo group. Per adjudication, the primary cause of death was cardiovascular in both treatment groups (during TEAE period, 5 (0.3%) cardiovascular death in alirocumab group versus 5 (0.6%) in placebo group).

Ninety-four (94) patients with a treatment emergent cardiovascular event were positively adjudicated (4.0% in alirocumab and 4.1% in placebo group). Table 4 summarizes any cardiovascular TEAEs according to adjudication. Table 5 summarizes adjudicated cardiovascular TEAEs, using the primary endpoint of the CVOT (ODYSSEY OUTCOMES) study (i.e. CHD death, non-fatal MI, fatal and non-fatal ischemic stroke, unstable angina requiring hospitalization).

Of note, at the time of the database lock, the adjudication process was still ongoing.

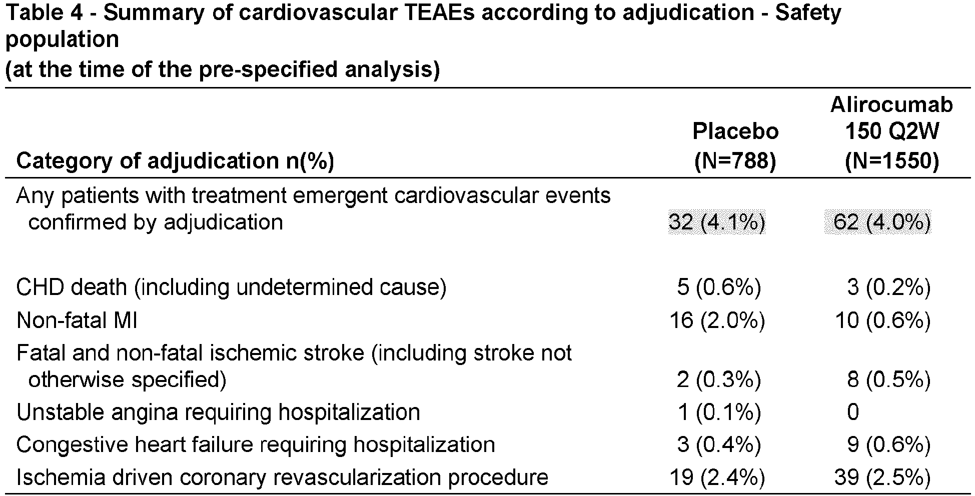

**Table 5 - Summary of cardiovascular TEAEs according to adjudication (CVOT study primary endpoint) - Safety population at the time of the pre-specified analysis)**

| **Category of adjudication n(%)** | **Placebo (N=788)** | **Alirocuma b 150 Q2W (N=1550)** |
|---|---|---|
| Any patients with treatment emergent cardiovascular events confirmed by adjudication (CVOT primary endpoint) | 24 (3.0%) | 22 (1.4%) |
| CHD death (including undetermined cause) | 6 (0.8%) | 3 (0.2%) |
| Non-fatal MI | 17 (2.2%) | 11 (0.7%) |
| Fatal and non-fatal ischemic stroke (including stroke not otherwise specified) | 2 (0.3%) | 8 (0.5%) |
| Unstable angina requiring hospitalization | 1 (0.1%) | 0 |

| | | |
|---|---|---|
| In a Cox model post-hoc analysis, the rate of adjudicated major cardiovascular events (cardiac death, myocardial infarction, ischemic stroke, and unstable angina requiring hospitalization) was 1.4% in the alirocumab arm versus 3.0% for placebo (nominal P= 0.0089), hazard ratio (HR) =0.46 (95% Cl: 0.26 to 0.82) (see Figure 4). | | |

Taken together, the interim safety analysis from this study showed fewer adjudicated major cardiovascular events in the alirocumab arm compared to placebo. In particular, there was an approximately 50 percent lower rate of adjudicated major CV events (cardiac death, myocardial infarction, stroke, and unstable angina requiring hospitalization) in the alirocumab group compared to placebo (1.4 percent compared to 3.0 percent, p<0.01) (see Table 5).

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

### Overall Summary of Pre-Specified Analysis

The ODYSSEY LONG TERM study described herein is the largest and longest double-blind study of a PCSK9 inhibitor. The current analysis provides about 1900 patient-years of double-blind patient exposure to alirocumab 150 mg Q2W. The following observations were made in high CV risk patients on maximally-tolerated statin ± other lipid lowering therapy: 1) self-administered alirocumab treatment produced significantly greater LDL-C reductions vs. placebo at Week 24 (LS mean difference -61.9%); 2) 79% of alirocumab patients achieved LDL-C goal of <0.81 mmol/L (70 mg/dL) at Week 24, regardless of risk; 81% of high-risk and very high-risk patients achieved their LDL-C goals (<100 mg/dL and <70 mg/dL, respectively); 3) mean achieved LDL-C levels of 1.4 mmol/L (53.1 mg/dL) at Week 52 with alirocumab; 4) lower rate of adjudicated major CV events observed in the alirocumab arm vs. placebo in a post-hoc analysis; 5) TEAEs occurred in a similar frequency in alirocumab and placebo arms; 6) treatment-emergent cardiovascular (CV) events were positively adjudicated in 4.0% and 4.4% of the alirocumab and placebo patients, respectively; and 7) a lower rate of adjudicated major CV events was observed in the alirocumab arm vs. placebo in a post-hoc analysis (HR=0.46, P<0.01).

### RESULTS II- FINAL SAFETY ANALYSIS

Although all of the primary and secondary efficacy endpoints were completed at the time of the pre-specified interim analysis, the study continued until completion, allowing analysis of the complete safety population.

### Summary safety population characteristics:

Mean study-drug exposure was 70 weeks in the 2338 patients included in the safety analysis (1550 in alirocumab and 788 in placebo groups), providing 2061 patient-years of exposure to alirocumab 150 mg every two weeks. Overall mean adherence to study treatment (i.e. the percentage of days that a patient took their injections as per the planned dosing schedule) was 98.0% and 97.6% in alirocumab and placebo groups, respectively. Mean duration of follow-up (regardless of treatment adherence) in the safety population was 80.9 weeks for alirocumab and 80.1 weeks for placebo. Study treatment discontinuation rates were 28% for alirocumab and 25% for placebo.

### Safety

Similar percentages of patients experienced treatment-emergent adverse events in both treatment groups (81% with alirocumab versus 83% with placebo) (Table 6).

Treatment-emergent adverse events leading to study-drug discontinuation occurred in 7.2% of alirocumab patients and 5.8% of placebo patients. With regard to specific adverse events, there were differences between the alirocumab and placebo groups in rates of injection-site reactions (5.9% versus 4.2%, respectively), myalgia (5.4% versus 2.9%, respectively), neurocognitive events (1.2% versus 0.5%, respectively), and ophthalmological events (2.9% versus 1.9%, respectively; Table 6).

Among the alirocumab patients, 575 (38% of the total) had two consecutive calculated LDL cholesterol levels of less than 25 mg/dL. Rates of treatment-emergent adverse events among these patients were comparable with those among the overall alirocumab group.

### Cardiovascular Events

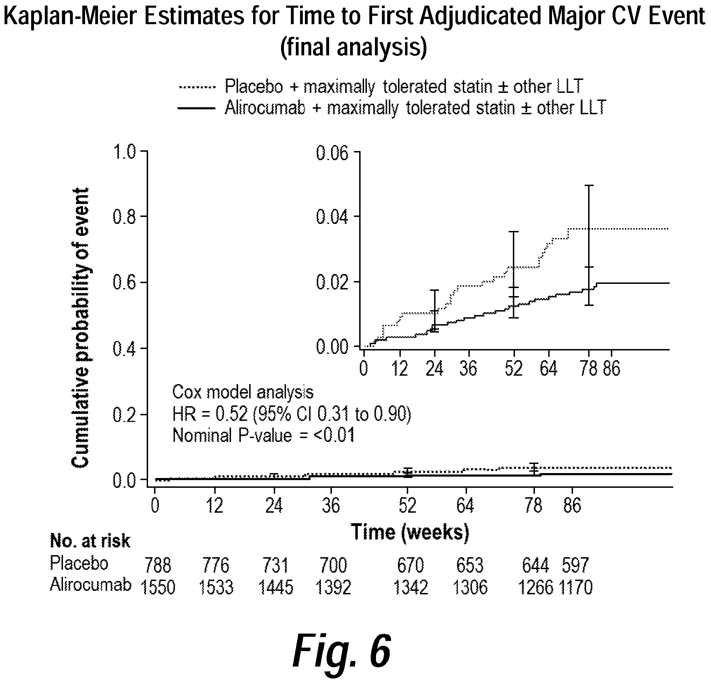

Positively adjudicated cardiovascular treatment-emergent adverse events occurred in 4.6% and 5.1% of alirocumab and placebo patients, respectively (Table 6). In a *post-hoc* analysis using the prespecified primary end point in the ongoing ODYSSEY OUTCOMES trial (coronary heart disease death, myocardial infarction, ischemic stroke, or unstable angina requiring hospitalization), a lower rate of adjudicated major adverse cardiac events was observed in the alirocumab group (27 of 1550 patients, 1.7%) compared with the placebo group (26 of 788 patients, 3.3%; hazard ratio 0.52; 95% Cl, 0.31 to 0.90; nominal P<0.01) (Table 3). The cumulative incidence curves diverged progressively over time (Figure 6). When all adjudicated cardiovascular events were included (adding congestive heart failure requiring hospitalization and ischemia-driven coronary revascularization), the difference between groups was not significant (Table 6).

**Table 6. AEs of Interest and Safety Laboratory Values (Safety Analysis)**

| **No. of patients** | | | **Alirocumab** | **Placebo** | **P-value** |
|---|---|---|---|---|---|
| Patients on maximally tolerated statin ± other LLT | | | (N=1550) | (N=788) | |

| **Summary of TEAEs*** | | | | | |
|---|---|---|---|---|---|
| | TEAEs | | 1255 (81.0) | 650 (82.5) | 0.3983 |
| | Treatment-emergent SAEs | | 290 (18.7) | 154 (19.5) | 0.6555 |
| | TEAEs leading to death | | 8 (0.5) | 10 (1.3) | 0.0760 |
| | TEAEs leading to discontinuation | | 111 (7.2) | 46 (5.8) | 0.2559 |

| **Cardiovascular AEs of interest** | | | | | |
|---|---|---|---|---|---|
| | CHD death including unknown cause | | 4 (0.3) | 7 (0.9) | 0.2559 |
| | Non-fatal Ml | | 14 (0.9) | 18 (2.3) | 0.0129 |
| | Fatal and non-fatal ischemic stroke | | 9 (0.6) | 2 (0.3) | 0.3528 |
| | Unstable angina requiring | | 0 | 1 (0.1) | 0.337 |
| | hospitalization | | | | |
| | Congestive heart failure requiring | | 9 (0.6) | 3 (0.4) | 0.761 |
| | hospitalization | | | | |
| | Ischemia-driven coronary | | 48 (3.1) | 24 (3.0) | 1 |
| | revascularization procedure | | | | |
| | Positively adjudicated cardiovascular events (including all cardiovascular AEs listed above) | | 72 (4.6) | 40 (5.1) | 0.682 |
| | *Post hoc* analysis of a subgroup of adjudicated MACE^{†} | | 27 (1.7) | 26 (3.3) | 0.0116 |

| **Other AEs of interest** | | | | | |
|---|---|---|---|---|---|
| | General allergic reaction events | | 156 (10.1) | 75 (9.5) | 0.7142 |
| | Local injection site reaction | | 91 (5.9) | 33 (4.2) | 0.0968 |
| | Myalgia | | 84 (5.4) | 23 (2.9) | 0.0063 |
| | Neurological events^{‡} | | 65 (4.2) | 35 (4.4) | 0.8289 |
| | Neurocognitive disorders^{§} | | 18 (1.2) | 4 (0.5) | 0.1727 |
| | | Amnesia | 5 (0.3) | 0 | 0.1747 |
| | | Memory impairment | 4 (0.3) | 1 (0.1) | 0.6686 |
| | | Confusional state | 4 (0.3) | 1 (0.1) | 0.6686 |
| | Ophthalmological events^{¶} | | 35 (2.9) | 15 (1.9) | 0.6516 |
| | Hemolytic anemia | | 0 | 0 | NC |
| | Diabetes in patients with no history of diabetes, n/N (%)" | | 18/994 (1.8) | 10/509 (2.0) | 0.8419 |
| | Worsening of diabetes in patients with history of diabetes, n/N (%)" | | 72/556 (12.9) | 38/279 (13.6) | 0.8284 |

| **Laboratory values of interest** | | | | | |
|---|---|---|---|---|---|
| | Alanine aminotransferase >3 times ULN, n/N (%) | | 28/1533 (1.8) | 16/779 (2.1) | 0.748 |
| | Aspartate aminotransferase >3 times ULN, n/N (%) | | 22/1533 (1.4) | 18/779 (2.3) | 0.1316 |
| | Creatine kinase >3 times ULN, n/N (%) | | 56/1507 (3.7) | 38/771 (4.9) | 0.1819 |

| | | | | | |
|---|---|---|---|---|---|
| P-values calculated using Fisher exact test and not adjusted for multiplicity. Provided for descriptive purpose only | | | | | |

### Overall Summary

At week 24, the difference between the alirocumab and placebo groups in mean calculated LDL cholesterol percent change from baseline was -62% (P<0.0001); the treatment effect remained consistent over 78 weeks with a 56 reduction from baseline in LDL-C for alirocumab versus placebo (p<0.001). There were differences between the alirocumab and placebo groups in rates of injection-site reactions (5.9% versus 4.2%, respectively), myalgia (5.4% versus 2.9%, respectively), neurocognitive events (1.2% versus 0.5%, respectively), and ophthalmological events (2.9% versus 1.9%, respectively). In a *post hoc* safety analysis, a significant reduction in cardiovascular events was seen with alirocumab, using the composite end point of coronary heart disease death, myocardial infarction, ischemic stroke or unstable angina requiring hospitalization, with a 48% reduction in the risk of major cardiovascular events over the 80 weeks of follow-up in 2341 patients.

### Example 4: Long-term Safety and Tolerability of Alirocumab in a Pooled Analysis of 5 Placebo-Controlled Phase 3 Clinical Trials

The ODYSSEY trials assessed the potential of subcutaneous alirocumab in one or more patient groups where there is high unmet need. One such population is patients with Heterozygous Familial hypercholesterolemia (HeFH), an inherited form of high cholesterol; ODYSSEY FH I, FH II and HIGH FH focused solely on patients in this group. HeFH is an inherited disorder of lipid metabolism that predisposes a person to high LDL-C and premature severe cardiovascular disease (CVD). A second population is patients with high or very high cardiovascular (CV) risk; ODYSSEY COMBO I, COMBO II, OPTIONS I, OPTIONS II and LONG TERM focused on these patients. A third population is patients with a history of statin-intolerance; ODYSSEY ALTERNATIVE included patients who had a history of being intolerant to statins and at moderate- to very-high CV risk.

The 9 ODYSSEY trials, along with the Odyssey MONO trial (addressing the effect of Alirocumab in the absence of any other lipid-modifying therapy), encompass over 5,000 patients studied in double-blind trials for 24-104 weeks. The trials evaluated two distinct dosing regimens: 150 milligrams (mg) every two weeks or 75 mg every two weeks increasing to 150 mg if needed to reach protocol-specified LDL-C targets. The 75 mg and the 150 mg dose were delivered with a single, self-administered one-milliliter (mL) injection. In each of these trials, Alirocumab was compared either to placebo or ezetimibe; thus, the trials can be grouped into placebo-controlled and ezetimibe-controlled trials.

### Adjudicated cardiovascular events

In all of the phase 3 studies, suspected CV events and all deaths that occurred from time of randomization until the follow-up visit were adjudicated by a clinical events committee. Analyses of the adjudicated events were performed on the pooled data from the 5 placebo-controlled trials. This analysis of the data from the pooled placebo-controlled studies are presented below with a primary focus on MACE events (CHD death, nonfatal MI, fatal or nonfatal ischemic stroke, and unstable angina requiring hospitalization). The MACE composite endpoint is generally considered the most appropriate and rigorous one to assess cardiovascular outcome and it is the primary endpoint of the OUTCOMES study (Example 2).

The phase 3 placebo-controlled pool combined five trials from the ODYSSEY program: LTS11717, FH I, FH II, HIGH FH, COMBO I. LTS11717 is described in detail above in Example 3. The design and rationale of FHI, FHII, and High FH are described in detail in Kastelein et al., Cardiovasc Drugs Ther. 2014; 28(3): 281-289. The design and rationale of Combo I is described in detail in Colhoun et al., BMC Cardiovasc Disord. 2014; 14: 121. These publications are hereby incorporated by reference in their entirety.

In the phase 3 placebo-controlled pool, the adjudicated composite MACE endpoint occurred in 35 (1.5%) patients in the alirocumab group (N=2318) and in 27 (2.3%) patients in the placebo group (N=1174). The incidence rates (per 100 patient-years) were 1.3 and 1.9 in the alirocumab and placebo groups, respectively, with a hazard ratio HR (95% Cl): 0.65 (0.40 to 1.08).

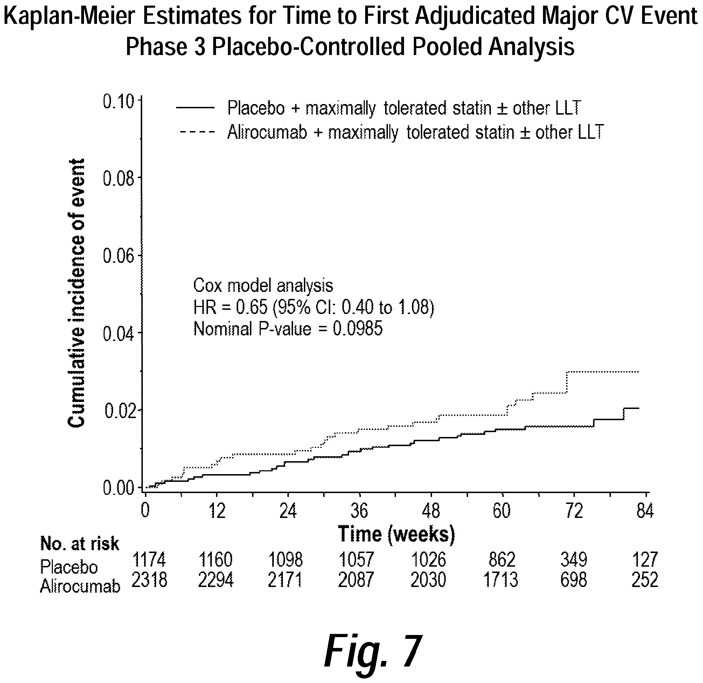

In the largest placebo-controlled study, LONG TERM (LTS11717), the adjudicated MACE composite event occurred in 22 (1.4%) patients in the alirocumab group (N=2318) and in 24 (3.0%) patients in the placebo group (HR: 0.46 [0.26 to 0.82]) (see also Example 3). Across the other placebo-controlled studies, a low number of MACE events was observed, leading to variable estimates of HR. Kaplan-Meier estimates for the time to first MACE endpoint are shown in Figure 7 (data censored on the TEAE period; last injection of study treatment + 70 days).

### Summary

Overall, in the placebo-controlled pool of phase 3 studies, a trend towards decrease of MACE events in the alirocumab arm when compared to placebo was observed, with an HR of 0.65 (95% Cl: 0.40 to 1.08), and particularly in the largest (n=2338) and longest (up to 18 months) single study LONG TERM, with an HR of 0.46 (95% Cl: 0.26 to 0.82). The majority of adjudicated and confirmed CV events were revascularizations and are not included in the MACE endpoint. Clinical standards for revascularization vary across the globe and it is likely that many of these cases reflect the greater attention to previous disease in the context of a clinical study.

The invention also pertains to the following:
1. A method for reducing cardiovascular risk in a high cardiovascular risk patient within 12 months following an acute coronary syndrome (ACS) event comprising administering one or more doses of a proprotein convertase subtilisin/kexin type 9 (PCSK9) inhibitor to the patient, wherein the patient exhibits inadequate control of atherogenic lipoproteins despite steady state treatment with a maximum tolerated dose statin therapy in the absence of the PCSK9 inhibitor.
2. The method of item 1, wherein reducing cardiovascular risk means reducing the time to first occurrence of coronary heart disease death, acute myocardial infarction, hospitalization for unstable angina, or ischemic stroke.
3. The method of item 1, wherein the ACS event is defined by: 1) unstable symptoms of myocardial ischemia occurring at rest or minimal exertion within 72 hours of an unscheduled hospital admission, due to presumed or proven obstructive coronary disease; and 2) at least one of the following: a) elevated cardiac biomarkers consistent with acute myocardial infarction, or b) resting ECG changes consistent with ischemia or infarction along with additional evidence of obstructive coronary disease from regional perfusion imaging or wall motion abnormalities, epicardial coronary stenosis ≥70% by angiography, or need for coronary revascularization related to the event.
4. The method of any one of items 1 to 3, wherein the PCSK9 inhibitor is an antibody or an antigen-binding fragment thereof that specifically binds PCSK9.
5. The method of item 4, wherein the antibody or antigen binding fragment thereof comprises the heavy and light chain complementarity determining regions (CDRs) of a heavy chain variable region/light chain variable region (HCVR/LCVR) amino acid sequence pair selected from the group consisting of SEQ ID NOs: 1/6 and 11/15.
6. The method of item 5, wherein the antibody or antigen-binding fragment thereof comprises heavy and light chain CDR amino acid sequences having SEQ ID NOs: 12, 13, 14, 16, 17, and 18.
7. The method of item 6, wherein the antibody or antigen-binding fragment thereof comprises an HCVR having the amino acid sequence of SEQ ID NO:11 and an LCVR having the amino acid sequence of SEQ ID NO: 15.
8. The method of item 5, wherein the antibody or antigen-binding fragment thereof comprises heavy and light chain CDR amino acid sequences having SEQ ID NOs: 2, 3, 4, 7, 8, and 10.
9. The method of item 8, wherein the antibody or antigen-binding fragment thereof comprises an HCVR having the amino acid sequence of SEQ ID NO:1 and an LCVR having the amino acid sequence of SEQ ID NO:6.
10. The method of item 4, wherein the antibody or antigen-binding fragment thereof binds to the same epitope on PCSK9 as an antibody comprising heavy and light chain CDR amino acid sequences having SEQ ID NOs: 12, 13, 14, 16, 17, and 18; or SEQ ID NOs: 2, 3, 4, 7, 8, and 10.
11. The method of item 4, wherein the antibody or antigen-binding fragment thereof competes for binding to PCSK9 with an antibody comprising heavy and light chain CDR amino acid sequences having SEQ ID NOs: 12, 13, 14, 16, 17, and 18; or SEQ ID NOs: 2, 3, 4, 7, 8, and 10.
12. The method of item 4, wherein the antibody or antigen-binding fragment thereof that specifically binds PCSK9 is administered to the patient at a dose of about 75 mg at a frequency of once every two weeks.
13. The method of item 12, wherein the about 75 mg dose is maintained if the patient's LDL-C measured after two doses is <50 mg/dL.
14. The method of item 12, wherein the about 75 mg dose is discontinued if the patient's LDL-C measured after two doses remains ≥50 mg/dL, and the antibody or antigen-binding fragment thereof that specifically binds PCSK9 is subsequently administered to the patient at a dose of about 150 mg at a frequency of once every two weeks.
15. The method of item 14, wherein the about 150 mg dose is discontinued if the patient's LDL-C for any two consecutive measurements is <25 mg/dL, and the antibody or antigen-binding fragment thereof that specifically binds PCSK9 is subsequently administered to the patient at a dose of about 75 mg at a frequency of once every two weeks.
16. The method of item 4, wherein the antibody or antigen-binding fragment thereof that specifically binds PCSK9 is administered to the patient at a dose of about 150 mg at a frequency of once every two weeks.
17. The method of any one of items 1 to 16, wherein the PCSK9 inhibitor is administered to the patient in combination with the maximum tolerated dose statin therapy.
18. The method of any one of items 1 to 17, wherein the maximum tolerated dose statin therapy comprises a daily dose of about 40 mg to about 80 mg of atorvastatin.
19. The method of any one of items 1 to 17, wherein the maximum tolerated dose statin therapy comprises a daily dose of about 20 mg to about 40 mg of rosuvastatin.
20. The method of any one of items 1 to 19, wherein the patient, prior to or at the time of administration of the PCSK9 inhibitor, exhibits inadequate control of atherogenic lipoproteins defined as: 1) a serum low-density lipoprotein cholesterol (LDL-C) level of ≥70 mg/dL; 2) non-high-density lipoprotein cholesterol ≥100 mg/dL; or 3) apolipoprotein B ≥80 mg/dL.
21. The method of any one of items 1 to 20, wherein the steady state treatment is treatment for at least two weeks.
22. A method for reducing cardiovascular events in a high cardiovascular risk patient within 12 months following an acute coronary syndrome (ACS) event comprising administering one or more doses of a proprotein convertase subtilisin/kexin type 9 (PCSK9) inhibitor to the patient, wherein the patient exhibits inadequate control of atherogenic lipoproteins despite steady state treatment with a maximum tolerated dose statin therapy in the absence of the PCSK9 inhibitor.
23. A method for reducing cardiovascular events in a high cardiovascular risk patient following an acute coronary syndrome (ACS) event comprising administering one or more doses of a proprotein convertase subtilisin/kexin type 9 (PCSK9) inhibitor to the patient.
24. A method for reducing cardiovascular risk in a high cardiovascular risk patient following an acute coronary syndrome (ACS) event comprising administering one or more doses of a proprotein convertase subtilisin/kexin type 9 (PCSK9) inhibitor to the patient.
25.The method of item 24, wherein reducing cardiovascular risk means reducing the time to first occurrence of coronary heart disease death, acute myocardial infarction, hospitalization for unstable angina, or ischemic stroke.
26. The method of any one of items 22-24, wherein the ACS event is defined by: 1) unstable symptoms of myocardial ischemia occurring at rest or minimal exertion within 72 hours of an unscheduled hospital admission, due to presumed or proven obstructive coronary disease; and 2) at least one of the following: a) elevated cardiac biomarkers consistent with acute myocardial infarction, or b) resting ECG changes consistent with ischemia or infarction along with additional evidence of obstructive coronary disease from regional perfusion imaging or wall motion abnormalities, epicardial coronary stenosis ≥70% by angiography, or need for coronary revascularization related to the event.
27. The method of any one of items 22 to 26, wherein the PCSK9 inhibitor is an antibody or an antigen-binding fragment thereof that specifically binds PCSK9.
28. The method of item 27, wherein the antibody or antigen binding fragment thereof comprises the heavy and light chain complementarity determining regions (CDRs) of a heavy chain variable region/light chain variable region (HCVR/LCVR) amino acid sequence pair selected from the group consisting of SEQ ID NOs: 1/6 and 11/15.
29. The method of item 28, wherein the antibody or antigen-binding fragment thereof comprises heavy and light chain CDR amino acid sequences having SEQ ID NOs: 12, 13, 14, 16, 17, and 18.
30. The method of item 29, wherein the antibody or antigen-binding fragment thereof comprises an HCVR having the amino acid sequence of SEQ ID NO:11 and an LCVR having the amino acid sequence of SEQ ID NO:15.
31. The method of item 28, wherein the antibody or antigen-binding fragment thereof comprises heavy and light chain CDR amino acid sequences having SEQ ID NOs: 2, 3, 4, 7, 8, and 10.
32. The method of item 31, wherein the antibody or antigen-binding fragment thereof comprises an HCVR having the amino acid sequence of SEQ ID NO:1 and an LCVR having the amino acid sequence of SEQ ID NO:6.
33. The method of item 4, wherein the antibody or antigen-binding fragment thereof binds to the same epitope on PCSK9 as an antibody comprising heavy and light chain CDR amino acid sequences having SEQ ID NOs: 12, 13, 14, 16, 17, and 18; or SEQ ID NOs: 2, 3, 4, 7, 8, and 10.
34. The method of item 27, wherein the antibody or antigen-binding fragment thereof competes for binding to PCSK9 with an antibody comprising heavy and light chain CDR amino acid sequences having SEQ ID NOs: 12, 13, 14, 16, 17, and 18; or SEQ ID NOs: 2, 3, 4, 7, 8, and 10.
35. The method of item 27, wherein the antibody or antigen-binding fragment thereof that specifically binds PCSK9 is administered to the patient at a dose of about 75 mg at a frequency of once every two weeks.
36. The method of item 35, wherein the about 75 mg dose is maintained if the patient's LDL-C measured after two doses is <50 mg/dL.
37. The method of item 35, wherein the about 75 mg dose is discontinued if the patient's LDL-C measured after two doses remains ≥50 mg/dL, and the antibody or antigen-binding fragment thereof that specifically binds PCSK9 is subsequently administered to the patient at a dose of about 150 mg at a frequency of once every two weeks.
38. The method of item 37, wherein the about 150 mg dose is discontinued if the patient's LDL-C for any two consecutive measurements is <25 mg/dL, and the antibody or antigen-binding fragment thereof that specifically binds PCSK9 is subsequently administered to the patient at a dose of about 75 mg at a frequency of once every two weeks.
39. The method of item 27, wherein the antibody or antigen-binding fragment thereof that specifically binds PCSK9 is administered to the patient at a dose of about 150 mg at a frequency of once every two weeks.
40. The method of any one of items 22 to 39, wherein the PCSK9 inhibitor is administered to the patient in combination with the maximum tolerated dose statin therapy.
41. The method of any one of items 22 to 40, wherein the maximum tolerated dose statin therapy comprises a daily dose of about 40 mg to about 80 mg of atorvastatin.
42. The method of any one of items 22 to 40, wherein the maximum tolerated dose statin therapy comprises a daily dose of about 20 mg to about 40 mg of rosuvastatin.
43. The method of any one of items 22 to 42, wherein the patient, prior to or at the time of administration of the PCSK9 inhibitor, exhibits inadequate control of atherogenic lipoproteins defined as: 1) a serum low-density lipoprotein cholesterol (LDL-C) level of ≥70 mg/dL; 2) non-high-density lipoprotein cholesterol ≥100 mg/dL; or 3) apolipoprotein B ≥80 mg/dL.
44. The method of any one of items 22 to 43, wherein the steady state treatment is treatment for at least two weeks.

## Claims

1. A pharmaceutical composition comprising an anti-PCSK9 (proprotein convertase subtilisin/kexin type 9) antibody or antigen-binding fragment thereof for use in increasing the time to first occurrence of coronary heart disease death, acute myocardial infarction, hospitalization for unstable angina, or ischemic stroke in a high cardiovascular risk patient within 12 months following an acute coronary syndrome (ACS) event,
wherein the antibody or antigen-binding fragment thereof has heavy and light chain CDR amino acid sequences having SEQ ID NOs: 2 (HCDR1), 3 (HCDR2), 4 (HCDR3), 7 (LCDR1), 8 (LCDR2), and 10 (LCDR3); and
wherein the antibody or antigen-binding fragment thereof is administered to the high cardiovascular risk patient at a dose of 75 mg at a frequency of once every two weeks,
wherein the 75 mg dose is maintained if the high cardiovascular risk patient's low-density lipoprotein cholesterol (LDL-C) measured after two doses is lower than 50 mg/dL, and
wherein the 75 mg dose is discontinued and the antibody or antigen-binding fragment thereof is subsequently administered to the high cardiovascular risk patient at a dose of 150 mg at a frequency of once every two weeks if the high cardiovascular risk patient's LDL-C measured after two doses is equal to or greater than 50 mg/dL;
wherein the high cardiovascular risk patient exhibits inadequate control of atherogenic lipoproteins despite steady state treatment with a maximum tolerated dose statin therapy,
wherein the high cardiovascular risk patient exhibiting inadequate control of atherogenic lipoproteins comprises:
1) a serum low-density lipoprotein cholesterol (LDL-C) level of ≥70 mg/dL;
2) non-high-density lipoprotein cholesterol≥100 mg/dL; or
3) apolipoprotein B≥80 mg/dL; and
wherein the maximum tolerated dose statin therapy comprises a daily dose of 40 mg to 80 mg of atorvastatin or a daily dose of 20 mg to 40 mg of rosuvastatin.

2. The pharmaceutical composition of claim 1, wherein the ACS event comprises:
1) unstable symptoms of myocardial ischemia occurring at rest or minimal exertion within 72 hours of an unscheduled hospital admission, due to presumed or proven obstructive coronary disease; and
2) at least one of the following:
a) elevated cardiac biomarkers consistent with acute myocardial infarction, or
b) resting ECG changes consistent with ischemia or infarction along with additional evidence of obstructive coronary disease from regional perfusion imaging or wall motion abnormalities, epicardial coronary stenosis≥70% by angiography, or need for coronary revascularization related to the event.

3. The pharmaceutical composition of claim 1 or 2, wherein the high cardiovascular risk patient has heterozygous familial hypercholesterolemia (heFH) or established coronary heart disease (CHD) or CHD risk equivalents.

4. The pharmaceutical composition of claim 3, wherein the high cardiovascular risk patient has a documented history of CHD, wherein the documented history of CHD is selected from the group consisting of:
(a) acute myocardial infarction (MI);
(b) silent myocardial infarction;
(c) unstable angina;
(d) coronary revascularization procedure; and
(e) clinically significant CHD.

5. The pharmaceutical composition of claim 3, wherein the high cardiovascular risk patient has CHD risk equivalents, wherein the CHD risk equivalents are selected from the group consisting of:
(a) peripheral arterial disease;
(b) previous ischemic stroke with a focal ischemic neurological deficit that persisted more than 24 hours, of atherothrombotic origin;
(c) moderate chronic kidney disease (CKD); and
(d) history of diabetes mellitus and two or more additional risk factors.

6. The pharmaceutical composition of any one of claims 1 to 5, wherein the antibody or antigen-binding fragment thereof comprises an HCVR having the amino acid sequence of SEQ ID NO: 1 and an LCVR having the amino acid sequence of SEQ ID NO:6.

7. The pharmaceutical composition of any one of claims 1 to 6, wherein the 150 mg dose is discontinued if the high cardiovascular risk patient's LDL-C for any two consecutive measurements is <25 mg/dL, and the antibody or antigen-binding fragment thereof is subsequently administered to the high cardiovascular risk patient at a dose of 75 mg at a frequency of once every two weeks.

8. The pharmaceutical composition of any one of claims 1 to 7, wherein the antibody or antigen-binding fragment thereof is administered to the high cardiovascular risk patient in combination with the maximum tolerated dose statin therapy.

9. The pharmaceutical composition of any one of claims 1 to 8, wherein the steady state treatment is treatment for at least two weeks.

10. The pharmaceutical composition of any one of claims 1 to 9, wherein the antibody or antigen-binding fragment thereof is administered subcutaneously.
